# EUROPEAN PATENT SPECIFICATION

(11) **EP 4 217 382 B1**
(45) Date of publication and mention of the grant of the patent: **28.10.2026**
(21) Application number: 21873371.5
(22) Date of filing: 22.09.2021
(51) Int. Cl.: C07K 14/78, C07K 14/435, G01N 33/68

(54) **METHODS FOR USING COLLAGEN HYBRIDIZING PEPTIDES TO DETERMINE COLLAGEN CONTENT**
VERFAHREN ZUR VERWENDUNG VON KOLLAGENHYBRIDISIERUNGSPEPTIDEN ZUR BESTIMMUNG DES KOLLAGENGEHALTS
PROCÉDÉS D'UTILISATION DE PEPTIDES D'HYBRIDATION DE COLLAGÈNE POUR DÉTERMINER LA TENEUR EN COLLAGÈNE

(30) Priority: 22.09.2020 US 202063081505 P
(43) Date of publication of application: 02.08.2023
(73) Proprietor: 3Helix, Inc., Salt Lake City, UT 84108 (US)
(72) Inventor: LI, Yang, Salt Lake City, Utah 84108 (US); BENNINK, Lucas, Salt Lake City, Utah 84108 (US); KIRKNESS, Michael, Salt Lake City, Utah 84108 (US); YU, S. Michael, Salt Lake City, Utah 84108 (US); BURT, Alastair, Salt Lake City, Utah 84108 (US)
(74) Representative: Graf von Stosch Patentanwaltsgesellschaft mbH
(86) International application number: PCT/US2021/051594
(87) International publication number: WO 2022/066799

(56) References cited:
- WO-A1-2019/083842
- LIN ALLEN H. ET AL: "Microplate assay for denatured collagen using collagen hybridizing peptides", vol. 37, no. 2, 3 January 2019 (2019-01-03), US, pages 431 - 438, XP093185045, ISSN: 0736-0266, Retrieved from the Internet <URL:https://api.wiley.com/onlinelibrary/tdm/v1/articles/10.1002%2Fjor.24185> DOI: 10.1002/jor.24185
- HWANG JEONGMIN, HUANG YUFENG, BURWELL TIMOTHY J., PETERSON NORMAN C., CONNOR JANE, WEISS STEPHEN J., YU S. MICHAEL, LI YANG: "In Situ Imaging of Tissue Remodeling with Collagen Hybridizing Peptides", ACS NANO, AMERICAN CHEMICAL SOCIETY, US, vol. 11, no. 10, 24 October 2017 (2017-10-24), US , pages 9825 - 9835, XP055826101, ISSN: 1936-0851, DOI: 10.1021/acsnano.7b03150
- JARAMILLO CATALINA, GUTHERY STEPHEN L., LOWICHIK AMY, STODDARD GREGORY, KIM TAEGUN, LI YANG, JENSEN MICHAEL KYLE: "Quantitative Liver Fibrosis Using Collagen Hybridizing Peptide to Predict Native Liver Survival in Biliary Atresia: A Pilot Study", JOURNAL OF PEDIATRIC GASTROENTEROLOGY AND NUTRITION, LIPPINCOTT WILLIAMS WILKINS, INC., US, vol. 70, no. 1, 1 January 2020 (2020-01-01), US , pages 87 - 92, XP055928748, ISSN: 0277-2116, DOI: 10.1097/MPG.0000000000002505

## Description

### FIELD

The present disclosure relates to methods for quantifying damaged collagen and collagen content.

### BACKGROUND

The extracellular matrix (ECM) plays a fundamental role in the regulation of normal and pathological processes. The most abundantly expressed component found in the ECM is collagen. The triple helical structure of collagen is known to be highly conserved, however in certain pathological conditions the triple helical structure of collagen can be disrupted. In many instances, the severity of the condition can be correlated with the extent or amount of collagen disruption. Existing techniques for quantifying collagen disruption can take several days or weeks, or involve complex staining procedures. Furthermore, even if the techniques are performed correctly, a trained pathologist or technician may be needed to analyze the results, which can introduce inter- or intra-person variability in the analysis.

Lin et al., 2019 developed a microplate assay for quantifying denatured collagen by measuring the fluorescence of carboxyfluorescein bound collagen hybridizing peptides (F-CHP) (Lin et al., Microplate Assay for Denatured Collagen using Collagen Hybridizing Peptides, J Orthop Res. 2019; 37(2): 431-438). Hwang et al., 2017 found out that a designed peptide (collagen hybridizing peptide: CHP), which specifically hybridizes to the degraded, unfolded collagen chains, can be used to image degraded collagen and inform tissue remodeling activity in various tissues: Labeled with 5-carboxyfluorescein and biotin, CHPs enabled direct localization and quantification of collagen degradation in isolated tissues within pathologic states ranging from osteoarthritis and myocardial infarction to glomerulonephritis and pulmonary fibrosis, as well as in normal tissues during developmental programs associated with embryonic bone formation and skin aging (Hwang et al., In Situ Imaging of Tissue Remodeling with Collagen Hybridizing Peptides, ACS Nano. 2017; 11(10): 9825-9835).

Accordingly, there is a need for a fast, reliable, and/or automated technique that can determine the damaged collagen or the total collagen content in a sample.

### BRIEF DESCRIPTION OF THE DRAWINGS

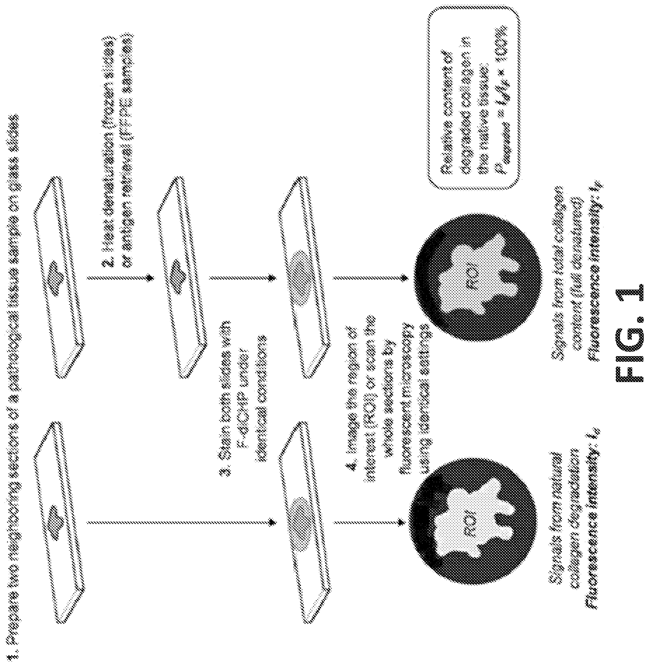

**FIG. 1** shows an exemplary process for determining denatured collagen content (left) and total collagen (right) allowing for the calculation of relative content of denatured (damaged/remodeling) collagen in a tissue sample treated with collagen hybridizing peptides (CHPs).

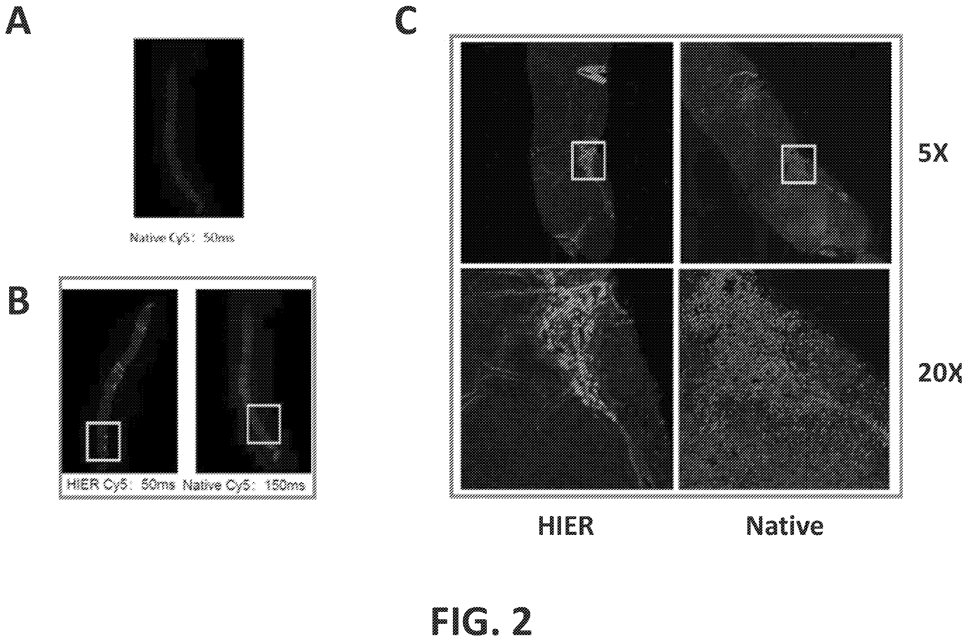

**FIG. 2A-C** shows immunohistochemistry images of fibrotic human liver tissue. (A) shows an image of healthy liver tissue without heat-induced epitope retrieval (HIER) followed by treatment with CHPs at 50 ms exposure time, which yields no observable collagen signal. (B) shows 1x magnification images of fibrotic liver tissue with HIER (left) and without HIER (right; Native) followed by treatment with CHPs. (C, top) shows 5x magnification images of the inset region of fibrotic liver tissue identified in panel B with (left) and without (right) HIER followed by treatment with CHPs. (C, bottom) shows 20x magnification images of the inset region of fibrotic liver tissue identified in the top row of panel C with (left) and without (right) HIER followed by treatment with CHPs. Samples processed with HIER show increased Cy5 signal corresponding to denatured collagen.

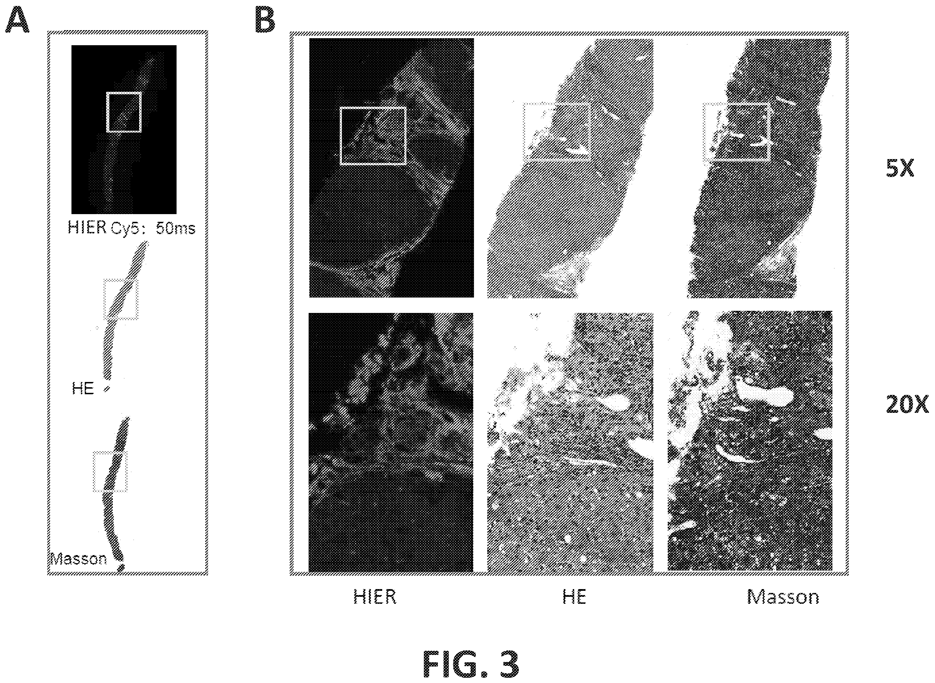

**FIG. 3A-B** shows immunohistochemistry images of human fibrotic liver tissue. (A) shows 1x magnification images of fibrotic liver tissue with HIER (top) followed by treatment with CHPs, fibrotic liver tissue stained with hematoxylin and eosin (HE), and fibrotic liver tissue stained with Masson's trichrome (Masson). (B, top) shows 5x magnification images of the inset region of fibrotic liver tissue identified in panel A with HIER followed by treatment with CHPs (left), with HE stain (middle), and with Masson's trichrome (right). (B, bottom) shows 20x magnification images of the inset region of fibrotic liver tissue identified in the top row of panel B with HIER followed by treatment with CHPs (left), with HE stain (middle), and with Masson's trichrome (right).

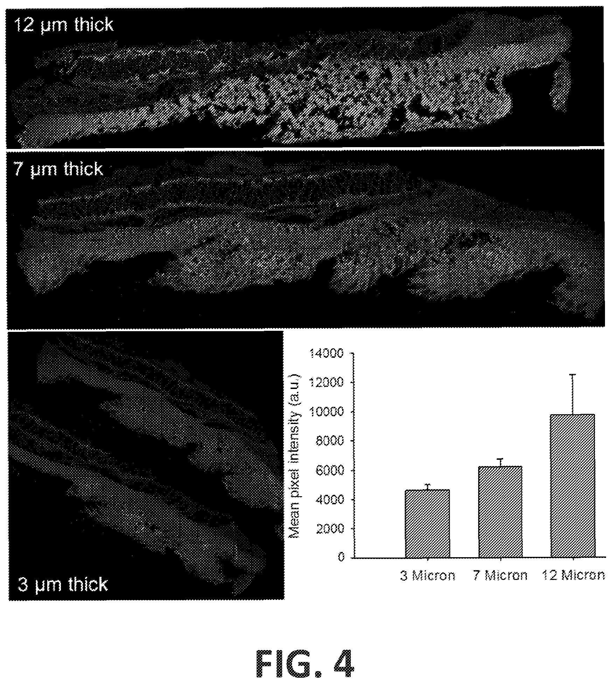

**FIG. 4** shows normal rabbit skin sections of different thickness stained with 20 µM R-CHP after heat induced epitope retrieval. As the thickness of the tissue sections increase, more CHP intensity is observed. Signal quantification using ImageJ/FIJI shows a near-linear correlation between tissue thickness and signal intensity.

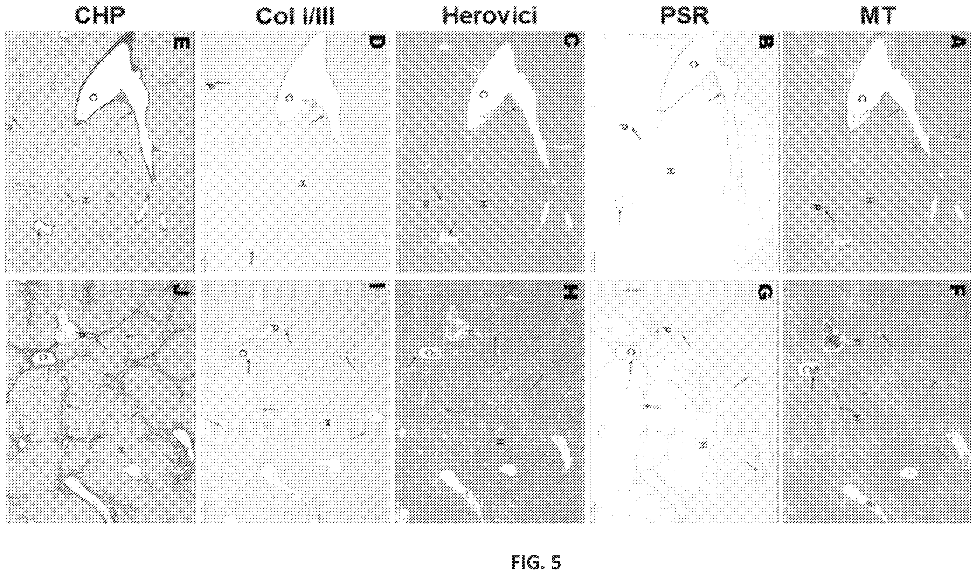

**FIG. 5** Representative photomicrographs of mouse livers stained using five different methods including Masson's Trichrome (MT), Herovici's Stain, Picrosirius red (PSR), collagen I & collagen III antibody cocktail (Col I/III), and biotin labeled CHPS (B-CHPs). Serial sections were taken from a healthy (control) mouse liver **(A-E)** and a fibrotic mouse liver 8 weeks after injection with CCl₄ **(F-J).** Collagen, identified by arrows in all photos, is stained blue in MT, pink/red in PSR, pink/red for mature collagen and blue for young collagen in Herovici's, dark to light brown for Col I/III cocktail, and dark brown in CHP staining (total collagen). Other features of interest are labeled as: C-central vein, H-hepatocytes, P-portal triads. Magnification was 40X, scale bar = 200 µM.

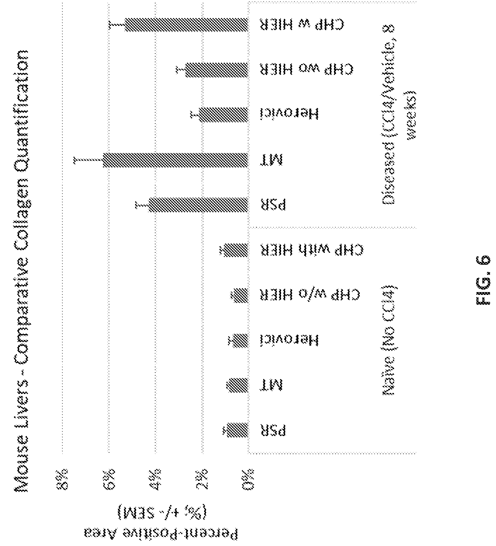

**FIG. 6** shows collagen quantification by image analysis software. Collagen was detected in all samples, with low overall collagen detected in naïve samples, and significantly higher quantities of collagen detected in diseased samples.

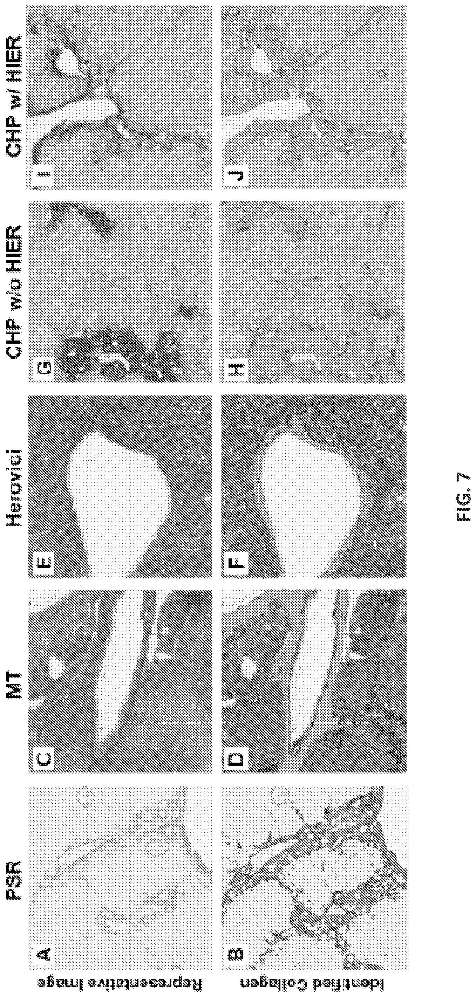

**FIG. 7** shows representative micrographs of detected collagen in fibrotic liver samples (CCl4, 8 weeks) using automated image analysis. In all images, green dotted lines represent the outer edge of the tissue and black dotted lines indicate areas excluded from analysis. Images in the top row are the images prior to analysis, and those in the bottom row show collagen highlighted by the image analysis (Red for PSR, bright green for all others).

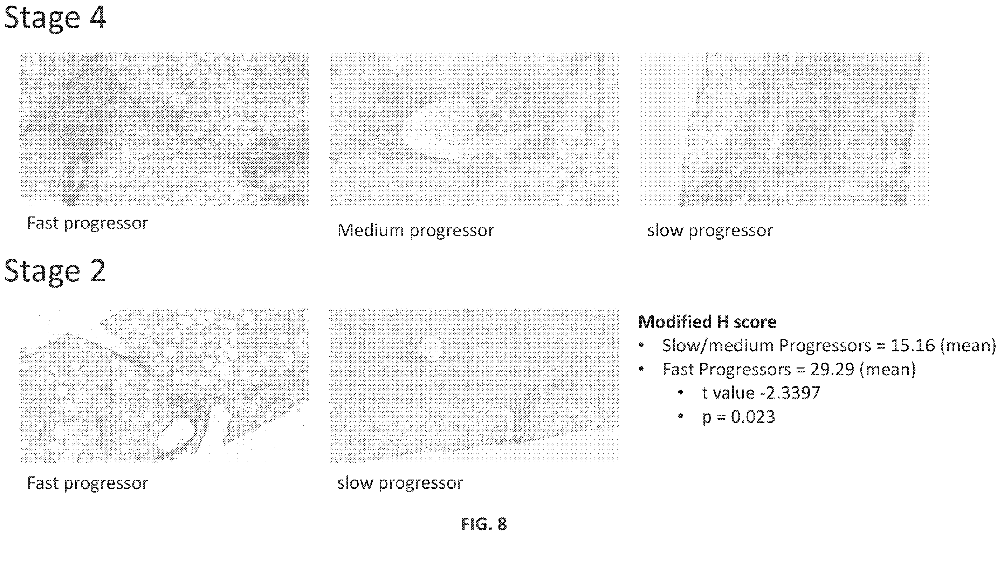

**FIG. 8** shows representative photographs of paired human liver biopsies stained using biotin labeled CHPs (B-CHPs). Subjects were scored by a pathologist of non-alcoholic fatty liver disease (NAFLD). B-CHPs were able to detect a difference in denatured collagen content due to disease remodeling in fast progressors versus the compiled medium, or slow progressing patients across all stages of NAFLD. These differences were found to be statistically significant with semi-quantitative assessment using modified H score which utilizes a multiplication of the area positivity x intensity.

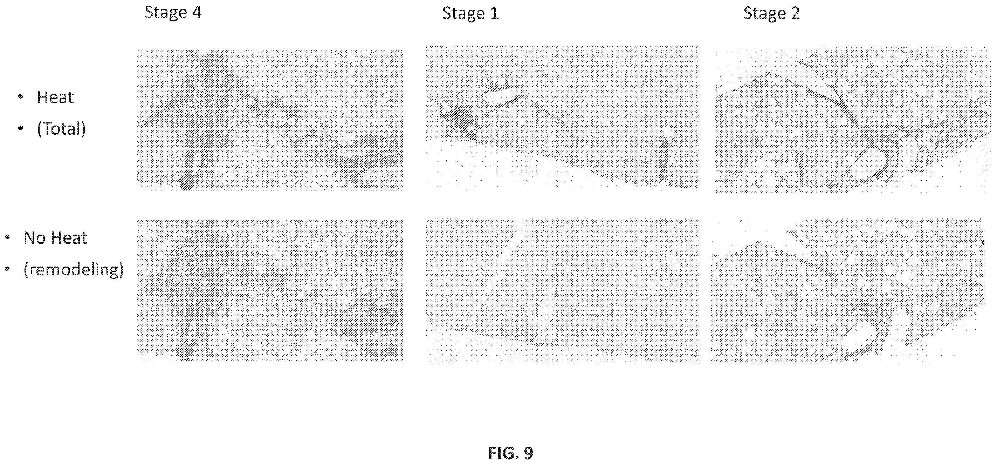

**FIG. 9** shows representative photographs showing total collagen (top row) and remodeled collagen from disease progression (bottom row) in B-CHP treated human liver biopsies. The paired liver biopsies were obtained from subjects across the NAFLD spectrum and were scored based on a pathologist's evaluation. Using total collagen staining via CHPs, pathologists were able differentiate between stage 1, 2, 3 and stage4, by calculating a ratio between the total collagen and the remodeled collagen stage1 with stage4 (p value = .00001) and stage2 with stage4 (p value = .237) and stage3 with stage4 (p value = 0.0197).

### DETAILED DESCRIPTION

### Overview

The present invention is defined in the appended claims. Evaluation of total collagen content can be performed by existing methods, for example, by using a trypsin-hydroxyproline assay. However, this method is cumbersome, as it can take up to several days to complete and is prone to error (*e.g*., from pipetting multiple times, which can compromise the results). Furthermore, this method completely destroys the sample so it cannot be used to provide spatial information about the distribution of denatured collagen within the sample. In contrast, certain methods described herein use collagen hybridizing peptides (CHPs) to quickly and easily determine the total collagen content within a tissue sample while also limiting destruction of the tissue. CHPs are disclosed, for example, in U.S. Patent Application Publication No. 2013/0164220. As shown in Lin et al., "Microplate assay for denatured collagen using collagen hybridizing peptides," J. Orthopaedic Research, 26 November 2018, CHPs can detect damaged or denatured collagen concentration with equal ability to trypsin-hydroxyproline assay.

The current gold standard for determining the severity of fibrosis in diseases such as non-alcoholic fatty liver disease (NAFLD), non-alcoholic steatohepatitis (NASH), chronic kidney disease (CKD), and idiopathic pulmonary fibrosis (IPF) is to take a biopsy and evaluate the tissues using histopathological or immunohistochemistry (IHC) techniques. Several stains can be used to identify collagens within a tissue biopsy (*e.g*., Picrosirius red (PSR)/Fast green, Masson's trichrome, and Herovici's Stain). Damaged collagen can be identified by the absence of stain or by colorimetric evaluation by a trained pathologist. In addition to the aforementioned stains being indirect indicators of collagen damage, these stains also require complex staining procedures with numerous steps, some require the use of specialized imaging equipment (polarized light), and no objective image analysis method for evaluating the collagen content. Trichrome staining (Masson's or Mallory's) has been found to underestimate the collagen content. This method utilizes three different dyes to stain cell nuclei, collagenous proteins, and cytoplasm. Intact collagen is stained blue or green, keratin and muscle fibers are stained red, the cytoplasm is stained pink, and the cell nuclei are brown or black. Damaged collagen is harder to distinguish as it will be a mixture between the available colors as the structure is damaged, therefore it is difficult for an objective image analysis method to identify the correct regions. Sirius Red/Fast Green stain was developed to overcome the problems with the trichrome stains and could offer better visualization by polarized light. This stains collagen fibers and bundles red while the non-collagenous proteins (*i.e.,* fibronectin, laminin) are stained green/yellow. This staining procedure utilizes polarized light to increase the specificity, sensitivity, and resolution. However, damaged collagens would appear as a dark region because damaged collagen lacks the orientation of intact collagen fibrils. Picrosirius red is an elongated birefringent molecule, and in tissues, picrosirius red binds to a variety of molecules, not just collagens. When bound to collagen, however, it orients parallel to the collagen fibrils, thereby greatly enhancing their natural birefringence. Thus, the complex fibrillar collagen/Sirius red is more birefringent than complexes made of Sirius red and other proteins. Sirius red-bound fibrillar collagens can then be detected under polarized light, where they appear bright and in sharp contrast with the rest of the tissue that remains dark/black. When the collagen is damaged it doesn't have birefringence. Lastly, Herovici's stain is capable of differentiating type I and type III collagen as well as young or old collagen. The young collagen is stained blue, mature collagen is stained red, cell nuclei are blue/black. However, it is subject to high variability in tissues where the collagen is denatured and the use of numerous colors of stains can produce variable results.

The evaluation of these stains can require a pathologist with a practiced eye to evaluate the coloration of the tissue, and each stain's coloring can be easily misinterpreted leading to inter- and intra-observer variations. This is partly due to the fact that damaged collagen is an unstructured protein and most stains or antibodies have difficulty targeting an unstructured 3D epitope, leading to regions that are partially stained or have incomplete staining. Therefore, when using these staining methods pathologists identify regions of damage collagen based on areas without stain (*e*.*g*., using Masson's Trichrome staining).

Another method for staining tissues is by utilizing antibodies for IHC. Even though antibodies are known to be highly specific, one of the main difficulties is overcoming specific or non-specific background which often times requires multiple blocking, washing, and antigen retrieval steps prior to application. Moreover, antibodies require a defined, intact, 3D epitope in order to bind successfully, so trying to bind to a damaged, unstructured protein such as denatured collagen, is often times impossible. One commercially available antibody is the C1,2C Antibody (Col 2 3/4C short Antibody) Rabbit Polyclonal Antibody (Ibex Pharmaceuticals; Montreal, CA) which can detect cleaved collagen type-I or -II segments. More specifically, it recognizes the α-chain fragments containing an approximate 8 amino acid sequence (GPPGPQG) at the carboxy terminus of the three-quarter piece produced by collagenase (MMP-1, MMP-8 and MMP-13) cleavage of type II collagen. However, due to chance of further cleavage by either MMPs or other enzymes can cleave the peptide before the antibody can bind because of the fast cleavage kinetics of enzymes, and clearance of short fragments from the area, detecting this fragment is inconsistent and does not convey the full extent of the damage. Because the CHPs in the methods of the present disclosure recognize the α-chain secondary structural motif, they can hybridize to any section with a sequence of (Gly-X-Y)₃ or longer (e.g., (Gly-X-Y)₄). Additionally, the CHPs described herein can hybridize with any collagen type (*i.e.,* Type I, II, III, IV, etc.) regardless of mechanism of damage (*i.e.,* thermal, mechanical, chemical, or enzymatic) making it far superior for the detection of collagen damage within a tissue section.

Using CHPs directly addresses issues with existing techniques. In one aspect, the present disclosure provides CHP-based methods that directly stain damaged collagen (of any type) in a single step, as a single stain, with high specificity and provides an objective imaging analysis method to quantify the regions of damaged collagen or to determine total collagen content without the need to evaluate coloration of the tissue stains. Biotin-labeled CHPs may be used with a light microscope (*e*.*g*., the current instrumentation used by pathologists for imaging and scoring stained samples) or a fluorescent microscope to read fluorescence intensity. Furthermore, CHP-based methods may use a single staining reagent, as compared to the multiple steps and stains required for other staining methods (*e*.*g*., Masson's trichrome).

In another aspect, methods of the present disclosure stain both damaged and total collagen in a heat-mediated staining process, thereby avoiding incomplete or partial staining at the interface of intact and damaged collagens as seen with alternative staining methods (*e*.*g*., Picrosirius red). The methods of the present disclosure show how CHPs may be used to stain for the total collagen content in the tissue sections and evaluate their fluorescence intensity using software (*e*.*g*., ImageJ). In certain embodiments, physicians may monitor the progression of fibrotic conditions by comparing the total collagen content at various time points during disease progression. In certain embodiments, methods of the present disclosure describe how to fully quantify all the collagen types within a tissue section using CHP staining techniques. In certain embodiments, CHPs are used to quickly and accurately identify the damaged collagen content within a tissue section, and determine the percent damage in the tissue (*e*.*g*., by using the total collagen content as 100% damaged).

### Definitions

As used herein "collagen" can be from any tissue type (e.g., bone, dermis, tendon, ligaments, etc.). Collagen can refer to a molecule in which three alpha chains of polyproline II-like structure fold together into a triple helix. Additionally, this can apply to any protein that contains a triple-helical region including collagen types I-XXVIII and bacterial collagen. The term "collagen" as used herein can refer to all forms of collagen, including artificial collagen and collagen which has been processed or otherwise modified. In some embodiments, the collagen is selected from type I collagen, type II collagen, type III collagen, type IV collagen, type V collagen, type VI collagen, type VII collagen, type VIII collagen, type IX collagen, type X collagen, type XI collagen, type XII collagen, type XIII collagen, type XIV collagen, type XV collagen, type XVI collagen, type XVII collagen, type XVIII collagen, type XIX collagen, type XX collagen, type XXI collagen, type XXII collagen, type XXIII collagen, type XXIV collagen, type XXV collagen, type XXVI collagen, type XXVII collagen, type XXVIII collagen, and a combination thereof.

As used herein "collagen content" can refer to an amount of collagen in a collagen-containing sample (*e*.*g*., tissue). Collagen content can refer to the weight of collagen in a sample, the volume of collagen in the sample, the fraction of a specific type of collagen (*e*.*g*., disrupted or denatured collagen) in a sample relative to the total amount of collagen in the sample. In some embodiments, determining collagen content can comprise determining an amount of total collagen in a sample (*e*.*g*., by intentionally denaturing native collagen). In other embodiments, determining collagen content can comprise determining an amount of disrupted collagen in a sample (*e*.*g*., as a fraction of the total collagen in the sample). A "denatured" collagen refers to collagen that is no longer in a triple helical form.

As used herein, "sample" can refer to a portion of a biological organism. The sample can be a cell, tissue, organ, or body part. A sample can be taken or isolated from the biological organism (*i.e.,* ex vivo), *e.g.,* a tumor sample removed from a subject. Exemplary biological samples include a skin sample, a muscle sample, a skeletal sample (bone), a neuronal sample, a connective tissue sample, an organ tissue sample (*e*.*g*., brain, lungs, liver, bladder, kidneys, heart, stomach, intestines, etc.), a tumor sample, a cancerous sample, biological fluids (e.g., a serum sample or a urine sample), or the like. The term "sample" also includes a mixture of the above-mentioned samples. The term "sample" also includes untreated or pretreated (or pre- processed) biological samples. In some embodiments, a sample can comprise one or more cells from the subject. In some embodiments the sample is substantially or entirely intact (*e*.*g*., morphology similar to the tissue in vivo). In other embodiments, the sample may be processed (*e*.*g*., ground or homogenized into a solution). In yet another embodiment, the sample may be artificial or synthetic.

As used herein "collagen-containing sample" or "collagen-containing tissue" can refer to skin, muscle and the like which can be isolated from a mammalian body that contains collagen. The term "collagen-containing sample" also encompasses "synthetically" produced tissue (*e*.*g*., artificial tissue) in which collagen or, collagen containing material has been assembled or manufactured outside a body. "Collagen-containing sample" can also refer to a sample (*e.g.,* a tissue sample or a biological fluid such as serum or urine) which has been homogenized into a homogeneous solution.

As used herein "disrupted collagen" can refer to a collagen molecule or a matrix thereof in which three alpha chains of collagen proteins, at least partially, do not form a triple helix in regions where the sequence suggest it should fold. "Disrupted collagen" can refer to a collagen molecule or a matrix thereof in which the three alpha chains of collagen proteins are, at least partially, unwound. The disrupted collagen can be disrupted full-length collagen or a fragment of collagen. A fragment of collagen can be any collagen sequence shorter than a full-length collagen sequence. Fragments also can be of a size such that they do not possess significant native structure or possess regions without significant native triple helical form.

As used herein "denatured collagen" can refer to collagen that is no longer in its native triple helical form resulting from thermal, mechanical, chemical, enzymatic, acidity, or other effects. The denatured collagen can be denatured full-length collagen or a fragment of collagen. A fragment of collagen can be any collagen sequence shorter than a full-length collagen sequence. Fragments also can be of a size such that they do not possess significant native structure or possess regions without significant native triple helical form.

As used herein "collagen hybridizing peptide" or CHP can refer to as a peptide having the sequence Sₘ-(Gly-X-Y)₃₋₂₀, in which S is a spacer molecule and m is an integer from 0 to 10, Gly is glycine, and at least one of X and Y is proline, modified proline, and/or hydroxyproline. In one example, a CHP can be Sₘ-(Gly-X-Hyp)₉, wherein S is a spacer molecule and m is an integer from 0 to 10 X is proline or modified proline, Gly is glycine, and Hyp is hydroxyproline.

As used herein "spacer molecule" can refer to one or more molecules or amino acids which are linked to the detectable moiety and/or the CHP. In one embodiment, the spacer is any one or more amino acids or their derivatives designated as Sₘ, where m is an integer of 0 to 10.

In one aspect, the CHP may be labeled with a detectable moiety. As used herein, "detectable moiety" can refer to a molecule bound to a CHP that is capable of generating a detectable signal when the CHP is bound to a target. The detectable signal may be an optical signal that can be imaged using an optical imaging system. The optical signal may be caused by a change in optical intensity of one or more wavelengths of light. The detectable moiety can be directly or indirectly bound to, hybridized to, conjugated to, or covalently linked to the CHP. In some embodiments, the detectable moiety is a fluorescent molecule or a chemiluminescent molecule. The CHP can be detected optically via the detectable moiety. Coupling may be covalent or non-covalent (*e*.*g*., via ionic interactions, Van der Waals forces, etc.). Where covalent coupling is implemented, the detectable moiety may be coupled to the CHP via a linker. In some cases, the linker is cleavable, such as photo-cleavable (*e*.*g*., cleavable under ultra-violet light), chemically-cleavable (*e*.*g*., via a reducing agent, such as dithiothreitol (DTT), tris(2-carboxyethyl)phosphine (TCEP)) or enzymatically cleavable (*e*.*g*., via an esterase, lipase, peptidase or protease). In some embodiments, a labeled CHP may comprise the following formula I:

L-Sₘ-(Gly-X-Y)₃₋₂₀ (Formula I)

in which L is one or more detectable moieties; S is a spacer molecule and m is an integer from 0 to 10; Gly is glycine; and at least one of X and Y is proline, modified proline, and/or hydroxyproline. In some embodiments, m is not 0. In some embodiments, m is 1. In some embodiments, m is 2. In some embodiments, m is 3.

As used herein, the term "antigen" refers to a compound, composition, or substance that may be specifically bound by the products of specific humoral or cellular immunity, such as an antibody molecule or T-cell receptor. Antigens can be any type of molecule including, for example, haptens, simple intermediary metabolites, sugars (e.g., oligosaccharides), lipids, and hormones as well as macromolecules such as complex carbohydrates (e.g., polysaccharides), phospholipids, nucleic acids and proteins.

As used herein the term "fixed sample" refers to a sample that has been treated to help preserve the morphological and/or biochemical features of the cells and structures as they existed before the sample was obtained from the organism. A fixed sample may be prepared by contacting the sample with an aldehyde fixative such as formaldehyde, paraformaldehyde, or glutaraldehyde. A fixed sample may be prepared by contacting the sample with an alcohol-based fixative such as one including methanol, ethanol or acetic acid. Alternatively, a fixed sample may be prepared by contacting the sample with an oxidizing agent such as osmium tetraoxide or potassium permanganate, or a fixed sample may be prepared using a metallic based fixative such as mercuric chloride or picric acid. In a particular embodiment, a fixed sample is prepared by contacting a sample comprising cells with a neutral buffered formalin (NBF) solution, which is typically used as a solution that is from about 2% to about 6%, or about 3.7% formalin (10% formaldehyde and 1% methanol).

As used herein, the term "slide" refers to any substrate (e.g., substrates made, in whole or in part, with glass, quartz, plastic, silicon, etc.) of any suitable dimensions on which a biological specimen is placed for analysis, and more particularly to a "microscope slide" such as a standard 3 inch (7.62 cm) by 1 inch (2.54 cm) microscope slide or a standard 75 mm by 25 mm microscope slide.

As used herein, the singular terms "a," "an," and "the" include plural referents unless the context clearly indicates otherwise. Similarly, the word "or" is intended to include "and" unless the context clearly indicates otherwise.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non- limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

The terms "comprising," "including," "having," and the like are used interchangeably and have the same meaning. Similarly, "comprises," "includes," "has," and the like are used interchangeably and have the same meaning. Specifically, each of the terms is therefore interpreted to be an open term meaning "at least the following," and is also interpreted not to exclude additional features, limitations, aspects, etc. Thus, for example, "a device having components a, b, and c" means that the device includes at least components a, b and c. Similarly, the phrase: "a method involving steps a, b, and c" means that the method includes at least steps a, b, and c. Moreover, while the steps and processes may be outlined herein in a particular order, the skilled artisan will recognize that the ordering steps and processes may vary unless a particular order is clearly indicated by the context.

As used herein, the term "about" refers to a numeric value, including, for example, whole numbers, fractions, and percentages, whether or not explicitly indicated. The term "about" generally refers to a range of numerical values (e.g., +/- 5, 6, 7, 8, 9 or 10% of the recited value) that one of ordinary skill in the art would consider equivalent to the recited value (e.g., having the same function or result). In some instances, the term "about" may include numerical values that are rounded to the nearest significant figure.

As used herein, the term "substantially" means the qualitative condition of exhibiting total or near-total extent or degree of a characteristic or property of interest. In some embodiments, "substantially" means within about 20%. In some embodiments, "substantially" means within about 15%. In some embodiments, "substantially" means within about 10%. In some embodiments, "substantially" means within about 5%.

### Collagen Hybridizing Peptides (CHPs) and Methods of Using the Same

Collagen is the major structural protein found in almost all human tissues. Collagen turnover is required for natural tissue homeostasis, therefore denatured collagen is present at basal levels. However, excessive collagen remodeling (both degradation or synthesis) is often associated with many human diseases and injuries. In certain embodiments, CHPs comprise a short repeating tripeptide that is capable of specifically binding to denatured, degraded, remodeling, or unwound collagen with no affinity for intact collagen molecules. CHPs can distinguish damaged collagen by recognizing a structural motif (*e*.*g*., the poly-proline II-like helix of alpha chains) which are not available on intact collagen molecules, then CHPs re-form the collagen triple helix through hybridization by forming a highly stable and long-lasting bond. In certain embodiments, the preset disclosure provides a method by which CHPs are used to directly determine the total collagen content as well as the percent of damaged collagen within tissues based on their total collagen content.

Fig. 1 shows an exemplary method of the present disclosure for estimating the content of degraded collagen in a pathological tissue specimen using CHPs. Fig. 1 illustrates a schematic of an exemplary process for how CHPs can be used to find the total amount of collagen (right) with heat denaturation, and subsequently determine the fraction of degraded collagen in the sample as a function of the total amount of collagen. Briefly, to determine the amount of disrupted collagen, a tissue sample is prepared on glass and stained with CHPs, which localize at regions of disrupted collagen. The tissue sample is imaged, for example, using fluorescence microscopy, and the fluorescence intensity is measured (Fig. 1, left). To determine the amount of total collagen, a serial tissue section or sample is prepared on glass and subjected to heat denaturation and/or antigen retrieval (*e*.*g*., heat induced epitope retrieval; HIER). Subsequently, the sample is processed identically to the sample in which disrupted collagen is being measured. Specifically, the sample is stained with CHPs and imaged (Fig. 1, right). Because the sample has been processed using HIER, the collagen in the sample is entirely disrupted, allowing the CHPs to bind and provide an optical signal corresponding to the total collagen in the sample.

Fig. 2A-C shows immunohistochemistry images of fibrotic human liver tissue. Methods of the present disclosure can be useful for researchers evaluating fibrotic conditions because the more severe the condition is, the more total collagen that will be present, which can provide insight into disease progression or serve as an additional biomarker for diagnosis. The total amount of collagen can be found by staining with CHPs after HIER. There is significantly higher signal in HIER sample at 50 ms than the native (unheated; non-HIER) sample at 150 ms exposure. (A) shows an image of healthy liver tissue without heat-induced epitope retrieval (HIER) followed by treatment with CHPs at 50 ms exposure time, which yields no observable collagen signal. (B) shows 1x magnification images of fibrotic liver tissue with (left; HIER) and without (right; Native) HIER followed by treatment with CHPs. (C, top) shows 5x magnification images of the inset region of fibrotic liver tissue identified in panel B with (left) and without (right) HIER followed by treatment with CHPs. (C, bottom) shows 20x magnification images of the inset region of fibrotic liver tissue identified in the top row of panel C with (left) and without (right) HIER followed by treatment with CHPs. Samples processed with HIER show increased Cy5 signal corresponding to denatured collagen.

FIG. 3A-B shows a comparison of immunohistochemistry images of fibrotic liver tissue stained using a method of the present disclosure (HIER), hematoxylin and eosin (HE), or Masson's trichrome (Masson). (A) shows 1x magnification images of fibrotic liver tissue with HIER (top) followed by treatment with CHPs, fibrotic liver tissue stained with hematoxylin and eosin (HE), and fibrotic liver tissue stained with Masson's trichrome (Masson). (B, top) shows 5x magnification images of the inset region of fibrotic liver tissue identified in panel A with HIER followed by treatment with CHPs (left), fibrotic liver tissue identified in panel A with HE stain (middle), and fibrotic liver tissue identified in panel A with Masson's trichrome (right). (B, bottom) shows 20x magnification images of the inset region of fibrotic liver tissue identified in the top row of panel B with HIER followed by treatment with CHPs (left), fibrotic liver tissue identified in panel A with HE stain (middle), and fibrotic liver tissue identified in panel A with Masson's trichrome (right). Comparing CHP staining (HIER) with H&E and Masson's Trichrome staining, fibrotic collagen in the liver can be visualized more clearly and more easily with CHP staining (HIER).

In certain embodiments, methods of the present disclosure comprise CHP application during heat mediated antigen retrieval/ heat-induced epitope retrieval (HIER) methods which fully denature the collagen within the tissue sections. This method has been shown to be successful using healthy rabbit skin tissues that vary in thickness, with results showing a near-linear correlation between collagen content and CHP signal intensity (Fig. 4).

In certain aspects, the present disclosure provides a method of measuring total collagen content in a sample according to claim 1. Generally, total collagen content can refer to the total amount of collagen in a collagen-containing sample (*e*.*g*., tissue). Collagen content can refer to the weight of collagen in a sample, the volume of collagen in the sample, the fraction of a specific type of collagen (*e*.*g*., disrupted or denatured collagen) in a sample relative to the total amount of collagen in the sample. According to the invention, determining collagen content comprises determining an amount of total collagen in a sample (*e*.*g*., by intentionally denaturing native collagen). Disclosed herein is also determining collagen content which comprises determining an amount of disrupted collagen in a sample (*e*.*g*., as a fraction of the total collagen in the sample).

In some embodiments, a tissue sample is obtained from a subject with a condition. In some embodiments, the condition is selected from the group consisting of glycogen storage disease type III (GSD II), glycogen storage disease type VI (GSD VI), glycogen storage disease type IX (GSD IX), non-alcoholic steatohepatitis (NASH), cirrhosis, hepatitis, scleroderma, alcoholic fatty liver disease, non-alcoholic fatty liver disease, atherosclerosis, asthma, fibrosis, cardiac fibrosis, organ transplant fibrosis, muscle fibrosis, pancreatic fibrosis, bone-marrow fibrosis, liver fibrosis, cirrhosis of liver and gallbladder, fibrosis of the spleen, kidney fibrosis, pulmonary fibrosis, idiopathic pulmonary fibrosis, diffuse parenchymal lung disease, idiopathic interstitial fibrosis, diffuse interstitial fibrosis, interstitial pneumonitis, desquamative interstitial pneumonia, respiratory bronchiolitis, interstitial lung disease, chronic interstitial lung disease, acute interstitial pneumonitis, hypersensitivity pneumonitis, nonspecific interstitial pneumonia, cryptogenic organizing pneumonia, lymphocytic interstitial pneumonia, pneumoconiosis, silicosis, emphysema, interstitial fibrosis, sarcoidosis, mediastinal fibrosis, cardiac fibrosis, atrial fibrosis, endomyocardial fibrosis, myocardial infarction, renal fibrosis, chronic kidney disease, Type II diabetes, macular degeneration, age-related macular degeneration, keloid lesions, hypertrophic scar, nephrogenic systemic fibrosis, injection fibrosis, complications of surgery, fibrotic chronic allograft vasculopathy and/or chronic rejection in transplanted organs, fibrosis associated with ischemic reperfusion injury, post-vasectomy pain syndrome, fibrosis associated with rheumatoid arthritis, arthrofibrosis, Dupuytren's disease, dermatomyositis-polymyositis, mixed connective tissue disease, fibrous proliferative lesions of the oral cavity, fibrosing intestinal strictures, Crohn's disease, glial scarring, leptomeningeal fibrosis, meningitis, systemic lupus erythematosus, fibrosis due to radiation exposure, fibrosis due to mammary cystic rupture, myelofibrosis, retroperitoneal fibrosis, progressive massive fibrosis, a wound, wound healing, aged skin, osteoarthritis, or symptoms or sequelae thereof, or other diseases or conditions resulting disruption of extracellular matrix components (e.g., disruption of the triple helicity of collagen).

In some embodiments, the sample comprises a tissue sample, and the tissue sample has a thickness of between about 1 micrometer (µm) and about 100 µm. In some embodiments, the sample comprises a tissue sample, and the tissue sample has a thickness of about 1 µm, about 2 µm, about 3 µm, about 4 µm, about 5 µm, about 6 µm, about 7 µm, about 8 µm, about 9 µm, about 10 µm, about 11 µm, about 12 µm, about 13 µm, about 14 µm, about 15 µm, about 16 µm, about 17 µm, about 18 µm, about 19 µm, about 20 µm, about 25 µm, about 30 µm, about 35 µm, about 40 µm, about 45 µm, about 50 µm, about 60 µm, about 70 µm about 80 µm, about 90 µm, about 100 µm, about 200 µm, about 300 µm, about 400 µm, about 500 µm, about 600 µm about 700 µm, about 800 µm, about 900 µm, about 1 millimeter (mm), about 1.2 mm, about 1.4 mm, about 1.6 mm, about 1.8 mm, about 2 mm, about 2.2 mm, about 2.4 mm, about 2.6 mm, about 2.8 mm, about 3 mm, about 4 mm, about 5 mm, or a range of any two values thereof.

As used herein, a "biological organism" or "subject" can refer to a human or an animal or bacteria or cell cultures from any of the aforementioned groups. Non-limiting examples of animals include vertebrates such as a primate, a rodent, a domestic animal, or a game animal. Primates include chimpanzees, cynomolgus monkeys, spider monkeys, and macaques (*e.g.*, Rhesus). Rodents include mice, rats, woodchucks, ferrets, rabbits, and hamsters. Domestic and game animals include cows, horses, pigs, deer, bison, buffalo, moose, feline species (*e*.*g*., domestic cat), and canine species (*e.g*., dog, fox, wolf). Fish including Chondrichthyes (cartilaginous fishes) and Osteichthyes (bony fishes). In certain embodiments, a biological organism or subject can refer to a zebrafish. The subject may be mammal. The mammal can be a human, non-human primate, mouse, rat, dog, cat, horse, or cow. In addition, the methods described herein can be used to diagnose and/or treat domesticated animals or pets. The term does not denote a particular age or sex. Thus, adult and newborn subjects, as well as fetuses, whether male or female, are intended to be included within the scope of this term.

A subject can be one who has been previously diagnosed with, identified as suffering from, and/or found to have a condition in need of treatment (*e*.*g*., fibrosis, wound / wound healing, idiopathic pulmonary fibrosis (IPF), aged skin, liver fibrosis, nonalcoholic steatohepatitis (NASH), nonalcoholic fatty liver disease (NAFLD), alcoholic fatty liver disease (AFLD), kidney fibrosis, myocardial infarction (MI), age-related macular degeneration (AMD), osteoarthritis (OA), keratoconus, or the like) or one or more complications related to the condition. In some embodiments, the condition is liver fibrosis. In some embodiments, the condition is nonalcoholic steatohepatitis (NASH). In some embodiments, the condition is nonalcoholic fatty liver disease (NAFLD). In some embodiments, the condition is alcoholic fatty liver disease (AFLD). The subject may optionally have already undergone treatment for the condition or the one or more complications related to the condition.

In another aspect of the invention, the methods described herein can be used to determine presence or progression of the condition in a patient as defined in claim 13. In some embodiments, the total collagen content and the damaged collagen content are combined as a ratio for an objective measure of damaged collagen that is normalized to the specific sample group. In some embodiments, the ratio is used as predictive biomarker of progression or resolution in a diseased state.

In some embodiments, the method described herein may further comprise detecting total collagen content in another sample from the patient. In some embodiments, the method described herein may further comprise detecting non-triple helical collagen in the same sample or another sample from the patient by contacting the labeled CHPs to the non-triple helical collagen. The non-triple helical collagen is detected by the same procedure as detecting the triple helical collagen, except that no heat is added to denature the collagen. In additional embodiments, the method further comprises comparing the detected collagen content (e.g., total collagen, non-triple helical collagen, or both) in a sample with that in said another sample or with a control content value.

Alternatively, a subject can be one who has not been previously diagnosed as having a condition or one or more complications related to the condition. For example, a subject can be one who exhibits one or more risk factors for a condition or one or more complications related to the condition. The subject may not exhibit risk factors. A "subject in need" of treatment for a particular condition can be a subject suspected of having that condition, diagnosed as having that condition, already treated or being treated for that condition, not treated for that condition, or at risk of developing that condition.

The methods and systems described herein can be used to image a sample from various subjects, including humans and nonhuman primates such as chimpanzees and other ape and monkey species; farm animals such as cattle, sheep, pigs, goats, and horses; domestic mammals such as dogs and cats; laboratory animals including rodents such as mice, rats, guinea pigs, and zebra fish; and the like. The sample may be isolated from a subject (*i.e.,* ex vivo). In other embodiments, the sample may be integral of a subject (*i.e.,* in vivo or in situ).

According to the invention, the method comprises denaturing collagen in the sample. It will be understood that any method known to a person of skill in the art may be used to denature a sample. Denaturing collagen in the sample can disrupt the triple helicity of the collagen in the sample.

Generally, the method may comprise denaturing collagen in the sample by antigen retrieval to produce a denatured sample. For example, the retrieval agent may be an antigen retrieval agent which includes one or more of water, a buffer, an enzyme, a chaotropic reagent, a chelating agent, a nucleophile, an oxidizing agent, an organic acid/base pair, an electron-deficient compound such as a Lewis acid, and a surfactant. The antigen retrieval agent may include at least two of water, a buffer, an enzyme, a chaotropic reagent, a chelating agent, a nucleophile, an oxidizing agent, an organic acid/base pair, an electron-deficient compound such as a Lewis acid, and a surfactant. The antigen retrieval agent may include at least three of water, a buffer, an enzyme, a chaotropic reagent, a chelating agent, a nucleophile, an oxidizing agent, an organic acid/base pair, an electron-deficient compound such as a Lewis acid, and a surfactant. The antigen retrieval agent may include a buffer (e.g. TRIS) and has a pH ranging from about 6 to about 9.

According to the invention, the method comprises denaturing collagen in the sample by exposing the sample to heat. In some embodiments, the method comprises denaturing collagen in the sample by exposing the sample to microwaves. In some embodiments, the method comprises denaturing collagen in the sample by exposing the sample to ultrasound. In some embodiments, the sample is heated to between about 50 degrees Celsius (°C) and about 160 °C. In some embodiments, the sample is heated to about 50 °C, about 55 °C, about 60 °C, about 65 °C, about 70 °C, about 75 °C, about 80°C, about 85 °C, about 90 °C, about 95 °C, about 100 °C, about 105 °C, about 110 °C, about 115 °C, about 120 °C, about 125 °C, about 130 °C, about 135 °C, about 140 °C, about 145°C, about 150 °C, about 155 °C, about 160 °C, or a range between any two values thereof. When the sample is heating, it can be held under pressure to minimize evaporation of the antigen retrieval agent or mitigate or prevent the boiling of the antigen retrieval agent.

In some embodiments, the method comprises denaturing collagen in the sample by exposing the sample to heat, and the sample is heated for between about 10 seconds and about 45 minutes. In some embodiments, the method comprises denaturing collagen in the sample by exposing the sample to heat, and the sample is heated for about 10 seconds (s), about 20 s, about 30 s, about 40 s, about 50 s, about 1 minute (min), about 1.5 min, about 2 min, about 2.5 min, about 3 min, about 3.5 min, about 4 min, about 4.5 min, about 5 min, about 5.5 min, about 6 min, about 6.5 min, about 7 min, about 7.5 min, about 8 min, about 8.5 min, about 9 min, about 9.5 min, about 10 min, about 11 min, about 12 min, about 13 min, about 14 min, about 15 min, about 16 min, about 17 min, about 18 min, about 19 min, about 20 min, about 21 min, about 22 min, about 23 min, about 24 min, about 25 min, about 26 min, about 27 min, about 28 min, about 29 min, about 30 min, about 31 min, about 32 min, about 33 min, about 34 min, about 35 min, about 36 min, about 37 min, about 38 min, about 39 min, about 40 min, about 41 min, about 42 min, about 43 min, about 44 min, about 45 min or a range of any two values thereof.

In some embodiments, the method comprises denaturing collagen in the sample by exposing the sample to electromagnetic radiation. In some embodiments, the electromagnetic radiation has a wavelength of between about 10 nm and about 400 nm (UV radiation). In some embodiments, the electromagnetic radiation has a wavelength of between about 200 nm and about 400 nm. In some embodiments, the electromagnetic radiation has a wavelength of between about 250 nm and about 400 nm. In some embodiments, the electromagnetic radiation includes one or more of UVA radiation (having a wavelength ranging from about 315 nm to about 400 nm), UVB radiation (having a wavelength ranging from about 280 nm to about 315 nm), and UVC radiation (having a wavelength ranging from about 100 nm to about 280 nm). The exposure time may be from about 10 min to about 2 hours, from about 10 minutes to about one hour, or from about 20 minutes to about 45 minutes. The power may be from about 1 J/cm² up to about 25 J/cm², from about 5 J/cm² up to about 15 J/cm², or from about 9 J/cm² up to about 12 J/cm² for UVA and from about 100 mJ/cm² to about 1 J/cm², from about 200 mJ/cm² to about 500 mJ/cm², or from about 250 mJ/cm² to about 350 mJ/cm² for UVB.

In some embodiments, the method comprises denaturing collagen using a method as described above, and the method also comprises antigen retrieval. In some embodiments, the denaturing and the antigen retrieval are performed simultaneously. In some embodiments, the denaturing and the antigen retrieval are performed sequentially. In some embodiments, the denaturing is performed before the antigen retrieval. In some embodiments, the denaturing is performed after the antigen retrieval.

According to the invention, the method comprises denaturing collagen using a method as described above, and the method also comprises contacting labeled collagen hybridizing peptides (CHPs) to the denatured collagen. In some embodiments, the denaturing and contacting labeled collagen hybridizing peptides (CHPs) to the denatured collagen are performed simultaneously. In some embodiments, the denaturing and contacting labeled collagen hybridizing peptides (CHPs) to the denatured collagen are performed sequentially. In some embodiments, the denaturing is performed before contacting labeled collagen hybridizing peptides (CHPs) to the denatured collagen. In some embodiments, the denaturing is performed after contacting labeled collagen hybridizing peptides (CHPs) to the sample (e.g., incubating the sample in a solution comprising CHPs, and then heating the sample to denature the collagen). As described herein, a method of the present disclosure can comprise contacting an undenatured sample with labeled collagen hybridizing peptides (CHPs), and simultaneously imaging and heating the sample to obtain (e.g., in real-time) a change in the fluorescence intensity corresponding to a difference between an amount of disrupted collagen in the native sample and the total amount of collagen.

According to the invention, when determining the total collagen content in a sample, the method comprises contacting labeled collagen hybridizing peptides (CHPs) to the denatured collagen (e.g., contacting the sample with CHPs after denaturing the collagen in the sample). In some embodiments, when determining the amount of disrupted collagen in the native sample, the method comprises contacting labeled collagen hybridizing peptides (CHPs) to the collagen in the native sample (e.g., contacting the sample with CHPs without denaturing the collagen in the sample).

Collagen content (e.g., total collagen content or an amount of disrupted collagen in the native sample) can be measured by detecting the intensity (or a change in intensity) of the optically detectable signal emitted from the label on the CHPs.

The methods and compositions disclosed herein make use of CHPs that include a detectable moiety (e.g., a label or tag). A label or tag allows for the detection of the labelled or tagged CHPs. A label or tag allows for the detection of disrupted collagen (e.g., CHP targets) in a sample when bound to a labelled or tagged CHPs. By label is meant a molecule that can be directly (i.e., a primary label) or indirectly (i.e., a secondary label) detected; for example, a label can be visualized and/or measured or otherwise identified so that its presence or absence can be known. A labelled or tagged CHP also allows for the detection of disrupted collagen (e.g., CHP targets) in a sample. A compound can be directly or indirectly conjugated to a label which provides a detectable signal, e.g. radioisotopes, fluorophores, enzymes, antibodies, particles such as magnetic particles, chemiluminescent compounds, or specific binding molecules, and the like. Examples of labels include, optical fluorescent and chromogenic dyes including labels, label enzymes and radioisotopes.

Examples of labels include: a) isotopic labels, which may be radioactive or heavy isotopes; b) magnetic labels, electrical labels, thermal labels; c) colored labels, optical labels including luminescent, phosphorous and fluorescent dyes or moieties; and d) binding partners. Labels also include enzymes (e.g. horseradish peroxidase, and the like.) and magnetic particles.

Labels include optical labels such as fluorescent dyes or moieties. Fluorophores are either "small molecule" fluors, or proteinaceous fluors (e.g. green fluorescent proteins and all variants thereof).

Suitable fluorescent labels include fluorescein, rhodamine, tetramethylrhodamine, eosin, erythrosin, coumarin, methyl-coumarins, pyrene, Malacite green, stilbene, Lucifer Yellow, Cascade Blue^{™}, Texas Red, IAEDANS, EDANS, BODIPY FL, LC Red 640, Cy 5, Cy 5.5, LC Red 705 and Oregon green. Suitable optical dyes are described in the 1996 Molecular Probes Handbook by Richard P. Haugland. Suitable fluorescent labels also include green fluorescent protein (GFP; Chalfie, et al., Science 263(5148):802-805, 1994); and EGFP; Clontech-Genbank Accession Number U55762), blue fluorescent protein (BFP; Quantum Biotechnologies, Inc.; Stauber, R. H. Biotechniques 24(3):462-471 (1998); Heim, R. and Tsien, R. Y. Curr. Biol. 6:178-182 (1996)), enhanced yellow fluorescent protein (EYFP; Clontech Laboratories, Inc.), luciferase (Ichiki, et al., J. Immunol. 150(12):5408-5417 (1993)), β-galactosidase (Nolan, et al., Proc Natl Acad Sci USA 85(8):2603-2607 (April 1988)) and Renilla (WO 92/15673; WO 95/07463; WO 98/14605; WO 98/26277; WO 99/49019; U.S. Pat. No. 5,292,658; U.S. Pat. No. 5,418,155; U.S. Pat. No. 5,683,888; U.S. Pat. No. 5,741,668; U.S. Pat. No. 5,777,079; U.S. Pat. No. 5,804,387; U.S. Pat. No. 5,874,304; U.S. Pat. No. 5,876,995; and U.S. Pat. No. 5,925,558).

In some embodiments, labels include: Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 660, Alexa Fluor 680), Cascade Blue, Cascade Yellow and R-phycoerythrin (PE) (Molecular Probes) (Eugene, Oreg.), FITC, Rhodamine, and Texas Red (Pierce, Rockford, Ill.), cyanine 3 (Cy3), Cy5, Cy5.5, Cy7, Cy7.5(Amersham Life Science, Pittsburgh, Pa.), Sulfo-Cyanine 3, Sulfo-Cyanine 5, Sulfo-Cyanine 5.5, Sulfo-Cyanine 7, Sulfo-Cyanine 7.5 (Lumiprobe, Hunt Valley, MD.). Tandem conjugate protocols for Cy5PE, Cy5.5PE, Cy7PE, Cy5.5APC, Cy7APC are known. Additional labels are available from commercial sources such as BD Biosciences, Beckman Coulter, AnaSpec, Invitrogen, Cell Signaling Technology, Millipore, eBioscience, Santa Cruz Biotech, Abcam, LiCor, and Sigma-Aldrich.

Fluorescent labels that are attached to CHPs can include dyes chosen for immunofluorescence that are excited by light of one wavelength (e.g., blue or green) and emit light of a different wavelength in the visible spectrum. The most common fluorescent dyes are fluorescein, which emits green light, Texas Red and Peridinn chlorophyll protein (PerCP), which emit red light, and rhodamine and phycoerythrin (PE) which emit orange/red light. By using selective filters, only the light coming from the dye or fluorochrome used is detected in the fluorescence microscope. This technique can be used to detect disrupted collagen (e.g., CHP targets) in a sample.

Confocal fluorescent microscopy, which uses computer-aided techniques to produce an ultrathin optical section of a cell or tissue, gives very high-resolution immunofluorescence microscopy without the need for elaborate sample preparation. The resolution of the confocal microscope can be further increased using low-intensity illumination so that two photons are required to excite the fluorochrome. A pulsed laser beam is used, and only when it is focused into the focal plane of the microscope is the intensity sufficient to excite fluorescence. In this way the fluorescence emission itself can be restricted to the optical section. In one embodiment, the use of a confocal fluorescent microscope allows for analysis of samples without any sample preparation.

One group of fluorophores that can be used as labels for CHPs of the present disclosure are xanthene dyes, which include the fluoresceins derived from 3,6-dihydroxy-9-henylxanthhydrol and resamines and rhodamines derived from 3,6-diamino-9-phenylxanthydrol and lissanime rhodamine B. The rhodamine and fluorescein derivatives of 9-o-carboxyphenylxanthhydrol have a 9-o-carboxyphenyl group. Fluorescein compounds having reactive coupling groups such as amino and isothiocyanate groups such as fluorescein isothiocyanate and fluorescamine are readily available. Another group of fluorescent compounds are the naphthylamines, having an amino group in the α- or β-position. In one aspect, CHPs are labeled with fluorochromes or chromophores by the procedures described by Goding, J. W. (Monoclonal Antibodies: Principles And Practice. New York: Academic Press (1983) pp 208-249).

In some embodiments, chemiluminescers such as luciferin are attached to the CHPs (See, e.g., U.S. Pat. No. 5,098,828, for synthesis and methods of detection).

In some embodiments, the CHPs comprises a secondary detectable label. A secondary label is one that is indirectly detected; for example, a secondary label can bind or react with a primary label for detection, can act on an additional product to generate a primary label (e.g. enzymes), and the like. Secondary labels include one of a binding partner pair; chemically modifiable moieties; nuclease inhibitors, enzymes such as horseradish peroxidase, alkaline phosphatases, luciferases, and the like.

In some embodiments, the secondary label is a binding partner pair. In one aspect, the label is a hapten or antigen, which will bind its binding partner. For example, suitable binding partner pairs include: antigens (such as proteins (including peptides) and small molecules) and antibodies (including fragments thereof (FAbs, and the like.)); proteins and small molecules, including biotin/streptavidin; enzymes and substrates or inhibitors; other protein-protein interacting pairs; receptor-ligands; and carbohydrates and their binding partners. Nucleic acid-nucleic acid binding protein pairs are contemplated. Binding partner pairs include biotin (or imino-biotin) and streptavidin, and digeoxinin and antibodies (Abs).

In some embodiment, the secondary label is a chemically modifiable moiety. In this embodiment, labels comprising reactive functional groups are incorporated into the molecule to be labeled. The functional group is then subsequently labeled (e.g. either before or after the assay) with a primary label. Suitable functional groups include amino groups, carboxy groups, maleimide groups, oxo groups and thiol groups. For example, primary labels containing amino groups are attached to secondary labels comprising amino groups, for example using known linkers; for example, homo- or hetero-bifunctional linkers.

In some embodiments, multiple fluorescent labels are employed in the methods and compositions disclosed herein. In some embodiments, each label is distinct and distinguishable from other labels.

In some embodiments, CHPs are labeled with quantum dots as disclosed by Chattopadhyay, P. K. et al. Quantum dot semiconductor nanocrystals for immunophenotyping by polychromatic flow cytometry. Nat. Med. 12, 972-977 (2006).

In some embodiments, the CHPs are labeled with tags suitable for Inductively Coupled Plasma Mass Spectrometer (ICP-MS) as disclosed in Tanner et al. Spectrochimica Acta Part B: Atomic Spectroscopy, 2007 March; 62(3):188-195; Ornatsky et al, Translational Oncogenomics (2006):1, 1-9; Ornatsky et al, Multiple Cellular Antigen Detection by ICP-MS, J. Imm. Methods 308 (2006) 68-76; and Lou et al., Polymer-Based Elemental Tags for Sensitive Bioassays, Angew. Chem. Int. Ed., (2007) 46, 6111-6114.

In one aspect, the CHPs comprises an enzyme label. By enzyme label is meant an enzyme that may be reacted in the presence of a label enzyme substrate that produces a detectable product. Enzyme labels include phosphatases or peroxidases covalently linked to a CHPs. Suitable enzyme labels include horseradish peroxidase, alkaline phosphatase and glucose oxidase. The presence of the enzyme label is generally revealed through the enzyme's catalysis of a reaction with a label enzyme substrate, producing an identifiable product that is detected and measured. The identifiable product may be a color change, detected with the naked eye or by a spectrophotometric technique, or the signal may be conversion of the substrate to a product that is detected by fluorescence. Such products may be opaque, such as the reaction of horseradish peroxidase with tetramethyl benzedine and may have a variety of colors. Other label enzyme substrates, such as Luminol (available from Pierce Chemical Co.), have been developed that produce fluorescent reaction products. Methods for identifying label enzymes with label enzyme substrates are well known in the art and many commercial kits are available. Examples and methods for the use of various label enzymes are described in Savage et al., Previews 247:6-9 (1998), Young, J. Virol. Methods 24:227-236 (1989).

In one aspect, the CHPs comprise a radioisotope/radiolabel. By radioisotope is meant any radioactive molecule. Suitable radioisotopes include 14C, 3H, 32P, 33P, 35S, 1251, 131I, 13N, 150, 18F, 57Co, 99mTc and 51Cr. The radiolabel is attached to the CHP by covalent linkage. In one aspect, CHPs include a radiolabel. In such a case scintillation counting is used. In such a case, samples that have been exposed to the radiolabeled CHPs are isolated and radioactivity of the bound ligands is measured.

In some embodiments, positron emitting isotopes detectable by a positron emission tomography ("PET") scanner are attached to a ligand. Examples of positron emitting isotopes include radioisotopes with short half-lives such as ¹¹C (^{~}20 min), ¹³N (^{~}10 min), ¹⁵O (^{~}2 min), and ¹⁸F (^{~}110 min). Methods for labeling of peptides with radioisotopes are known in the art. For example, such methods are found in Ohta et al., (1999) Molec. Cell 3:535-541. In some embodiments, CHPs are labeled with an NMR-active isotope label such as the ¹⁹F atom, or the ¹⁵N atom, or a plurality of such atoms.

Generally, a method of the present disclosure comprises using a CHP. In one embodiment, a CHP comprises a sequence represented by Formula I:

L-Sₘ-(Gly-X-Y)₃₋₂₀ (Formula I).

in which L is one or more detectable moieties (e.g., label); S is a spacer molecule and m is an integer from 0 to 10; and (Gly-X-Y)₃₋₂₀ represents a repeating portion in which Gly is glycine; and at least one of X and Y is proline, modified proline, and/or hydroxyproline. In some embodiments, a method of the present disclosure comprise using a CHP comprising a sequence represented by L-Sₘ-(Gly-X-Y)₃, a sequence represented by L-Sₘ-(Gly-X-Y)₄, a sequence represented by L-Sₘ-(Gly-X-Y)₅, a sequence represented by L-Sₘ-(Gly-X-Y)₆, a sequence represented by L-Sₘ-(Gly-X-Y)₇, a sequence represented by L-Sₘ-(Gly-X-Y)₈, a sequence represented by L-Sₘ-(Gly-X-Y)₉, a sequence represented by L-Sₘ-(Gly-X-Y)₁₀, a sequence represented by L-Sₘ-(Gly-X-Y)₁₁, a sequence represented by L-Sₘ-(Gly-X-Y)₁₂, a sequence represented by L-Sₘ-(Gly-X-Y)₁₃, a sequence represented by L-Sₘ-(Gly-X-Y)₁₄, a sequence represented by L-Sₘ-(Gly-X-Y)₁₅, a sequence represented by L-Sₘ-(Gly-X-Y)₁₆, a sequence represented by L-Sₘ-(Gly-X-Y)₁₇, a sequence represented by L-Sₘ-(Gly-X-Y)₁₈, a sequence represented by L-Sₘ-(Gly-X-Y)₁₉, or a sequence represented by L-Sₘ-(Gly-X-Y)₂₀, in which L is one or more detectable moieties (e.g., label); S is a spacer molecule and m is an integer from 0 to 10; Gly is glycine; and at least one of X and Y is proline, modified proline, and/or hydroxyproline. In some embodiments, m is not 0. In some embodiments, m is 1. In some embodiments, m is 2. In some embodiments, m is 3.

In some embodiments, a method of the present disclosure can comprise contacting a sample with a CHP having a sequence selected from SEQ ID NO.: 1 - 337 (see, e.g., Table 1).

**Table 1 -Sequences for Collagen Hybridizing Peptides**

| **Identifier** | **Sequence (One-Letter Amino Acid Symbols)** |
|---|---|
| SEQ ID NO:1 | Biotin-GGG-GPOGPOGPOGPOGPOGPOGPOGPOGPO-NH₂ |
| SEQ ID NO:2 | 5(6)-Carboxyfluorescein-GGG-GPOGPOGPOGPOGPOGPOGPOGPOGPO-NH₂ |
| SEQ ID NO:3 | Alexa Fluor-350-GGG-GPOGPOGPOGPOGPOGPOGPOGPOGPO-NH₂ |
| SEQ ID NO:4 | Alexa Fluor-430-GGG-GPOGPOGPOGPOGPOGPOGPOGPOGPO-NH₂ |
| SEQ ID NO:5 | Alexa Fluor-488-GGG-GPOGPOGPOGPOGPOGPOGPOGPOGPO-NH₂ |
| SEQ ID NO:6 | Alexa Fluor-546-GGG-GPOGPOGPOGPOGPOGPOGPOGPOGPO-NH₂ |
| SEQ ID NO:7 | Alexa Fluor-568-GGG-GPOGPOGPOGPOGPOGPOGPOGPOGPO-NH₂ |
| SEQ ID NO:8 | Alexa Fluor-594-GGG-GPOGPOGPOGPOGPOGPOGPOGPOGPO-NH₂ |
| SEQ ID NO:9 | Alexa Fluor-633-GGG-GPOGPOGPOGPOGPOGPOGPOGPOGPO-NH₂ |
| SEQ ID NO:10 | Alexa Fluor-660-GGG-GPOGPOGPOGPOGPOGPOGPOGPOGPO-NH₂ |
| SEQ ID NO:11 | Alexa Fluor-680-GGG-GPOGPOGPOGPOGPOGPOGPOGPOGPO-NH₂ |
| SEQ ID NO:12 | Cyanine 3-GGG-GPOGPOGPOGPOGPOGPOGPOGPOGPO-NH₂ |
| SEQ ID NO:13 | Cyanine 5-GGG-GPOGPOGPOGPOGPOGPOGPOGPOGPO-NH₂ |
| SEQ ID NO:14 | Cyanine 5.5-GGG-GPOGPOGPOGPOGPOGPOGPOGPOGPO-NH₂ |
| SEQ ID NO:15 | Cyanine 7-GGG-GPOGPOGPOGPOGPOGPOGPOGPOGPO-NH₂ |
| SEQ ID NO:16 | Cyanine 7.5-GGG-GPOGPOGPOGPOGPOGPOGPOGPOGPO-NH₂ |
| SEQ ID NO:17 | Cyanine 3-phycoerythrin-GGG-GPOGPOGPOGPOGPOGPOGPOGPOGPO-NH₂ |
| SEQ ID NO:18 | Cyanine 5-phycoerythrin-GGG-GPOGPOGPOGPOGPOGPOGPOGPOGPO-NHz |
| SEQ ID NO:19 | Cyanine 5.5-phycoerythrin-GGG-GPOGPOGPOGPOGPOGPOGPOGPOGPO-NH₂ |
| SEQ ID NO:20 | Cyanine 7-phycoerythrin-GGG-GPOGPOGPOGPOGPOGPOGPOGPOGPO-NH₂ |
| SEQ ID NO:21 | Cyanine 7.5-phycoerythrin-GGG-GPOGPOGPOGPOGPOGPOGPOGPOGPO-NH₂ |
| SEQ ID NO:22 | Rhodamine-GGG-GPOGPOGPOGPOGPOGPOGPOGPOGPO-NH₂ |
| SEQ ID NO:23 | Sulfo-Cyanine3-GGG-GPOGPOGPOGPOGPOGPOGPOGPOGPO-NH₂ |
| SEQ ID NO:24 | Sulfo-Cyanine5-GGG-GPOGPOGPOGPOGPOGPOGPOGPOGPO-NH₂ |
| SEQ ID NO:25 | Sulfo-Cyanine5.5-GGG-GPOGPOGPOGPOGPOGPOGPOGPOGPO-NH₂ |
| SEQ ID NO:26 | Sulfo-Cyanine7-GGG-GPOGPOGPOGPOGPOGPOGPOGPOGPO-NH₂ |
| SEQ ID NO:27 | Sulfo-Cyanine7.5-GGG-GPOGPOGPOGPOGPOGPOGPOGPOGPO-NH₂ |
| SEQ ID NO:28 | Texas Red-GGG-GPOGPOGPOGPOGPOGPOGPOGPOGPO-NH₂ |
| SEQ ID NO:29 | Biotin-GGG-GfOGfOGfOGfOGfOGfOGfOGfOGfO-NH₂ |
| SEQ ID NO:30 | 5(6)-Carboxyfluorescein-GGG-GfOGfOGfOGfOGfOGfOGfOGfOGfO-NH₂ |
| SEQ ID NO:31 | Alexa Fluor-350-GGG-GfOGfOGfOGfOGfOGfOGfOGfOGfO-NH₂ |
| SEQ ID NO:32 | Alexa Fluor-430-GGG-GfOGfOGfOGfOGfOGfOGfOGfOGfO-NH₂ |
| SEQ ID NO:33 | Alexa Fluor-488-GGG-GfOGfOGfOGfOGfOGfOGfOGfOGfO-NH₂ |
| SEQ ID NO:34 | Alexa Fluor-546-GGG-GfOGfOGfOGfOGfOGfOGfOGfOGfO-NH₂ |
| SEQ ID NO:35 | Alexa Fluor-568-GGG-GfOGfOGfOGfOGfOGfOGfOGfOGfO-NH₂ |
| SEQ ID NO:36 | Alexa Fluor-594-GGG-GfOGfOGfOGfOGfOGfOGfOGfOGfO-NH₂ |
| SEQ ID NO:37 | Alexa Fluor-633-GGG-GfOGfOGfOGfOGfOGfOGfOGfOGfO-NH₂ |
| SEQ ID NO:38 | Alexa Fluor-660-GGG-GfOGfOGfOGfOGfOGfOGfOGfOGfO-NH₂ |
| SEQ ID NO:39 | Alexa Fluor-680-GGG-GfOGfOGfOGfOGfOGfOGfOGfOGfO-NH₂ |
| SEQ ID NO:40 | Cyanine 3-GGG-GfOGfOGfOGfOGfOGfOGfOGfOGfO-NH₂ |
| SEQ ID NO:41 | Cyanine 5-GGG-GfOGfOGfOGfOGfOGfOGfOGfOGfO-NH₂ |
| SEQ ID NO:42 | Cyanine 5.5-GGG-GfOGfOGfOGfOGfOGfOGfOGfOGfO-NH₂ |
| SEQ ID NO:43 | Cyanine 7-GGG-GfOGfOGfOGfOGfOGfOGfOGfOGfO-NH₂ |
| SEQ ID NO:44 | Cyanine 7.5-GGG-GfOGfOGfOGfOGfOGfOGfOGfOGfO-NH₂ |
| SEQ ID NO:45 | Cyanine 3-phycoerythrin-GGG-GfOGfOGfOGfOGfOGfOGfOGfOGfO-NH₂ |
| SEQ ID NO:46 | Cyanine 5-phycoerythrin-GGG-GfOGfOGfOGfOGfOGfOGfOGfOGfO-NH₂ |
| SEQ ID NO:47 | Cyanine 5.5-phycoerythrin-GGG-GfOGfOGfOGfOGfOGfOGfOGfOGfO-NH₂ |
| SEQ ID NO:48 | Cyanine 7-phycoerythrin-GGG-GfOGfOGfOGfOGfOGfOGfOGfOGfO-NH₂ |
| SEQ ID NO:49 | Cyanine 7.5-phycoerythrin-GGG-GfOGfOGfOGfOGfOGfOGfOGfOGfO-NH₂ |
| SEQ ID NO:50 | Rhodamine-GGG-GfOGfOGfOGfOGfOGfOGfOGfOGfO-NH₂ |
| SEQ ID NO:51 | Sulfo-Cyanine3-GGG-GfOGfOGfOGfOGfOGfOGfOGfOGfO-NH₂ |
| SEQ ID NO:52 | Sulfo-Cyanine5-GGG-GfOGfOGfOGfOGfOGfOGfOGfOGfO-NH₂ |
| SEQ ID NO:53 | Sulfo-Cyanine5.5-GGG-GfOGfOGfOGfOGfOGfOGfOGfOGfO-NH₂ |
| SEQ ID NO:54 | Sulfo-Cyanine7-GGG-GfOGfOGfOGfOGfOGfOGfOGfOGfO-NH₂ |
| SEQ ID NO:55 | Sulfo-Cyanine7.5-GGG-GfOGfOGfOGfOGfOGfOGfOGfOGfO-NH₂ |
| SEQ ID NO:56 | Texas Red-GGG-GfOGfOGfOGfOGfOGfOGfOGfOGfO-NH₂ |
| SEQ ID NO:57 | Biotin-Ahx-GPOGPOGPOGPOGPOGPOGPOGPOGPO-NH₂ |
| SEQ ID NO:58 | 5(6)-Carboxyfluorescein-Ahx-GPOGPOGPOGPOGPOGPOGPOGPOGPO-NH₂ |
| SEQ ID NO:59 | Alexa Fluor-350-Ahx-GPOGPOGPOGPOGPOGPOGPOGPOGPO-NH₂ |
| SEQ ID NO:60 | Alexa Fluor-430-Ahx-GPOGPOGPOGPOGPOGPOGPOGPOGPO-NH₂ |
| SEQ ID NO:61 | Alexa Fluor-488-Ahx-GPOGPOGPOGPOGPOGPOGPOGPOGPO-NH₂ |
| SEQ ID NO:62 | Alexa Fluor-546-Ahx-GPOGPOGPOGPOGPOGPOGPOGPOGPO-NH₂ |
| SEQ ID NO:63 | Alexa Fluor-568-Ahx-GPOGPOGPOGPOGPOGPOGPOGPOGPO-NH₂ |
| SEQ ID NO:64 | Alexa Fluor-594-Ahx-GPOGPOGPOGPOGPOGPOGPOGPOGPO-NH₂ |
| SEQ ID NO:65 | Alexa Fluor-633-Ahx-GPOGPOGPOGPOGPOGPOGPOGPOGPO-NH₂ |
| SEQ ID NO:66 | Alexa Fluor-660-Ahx-GPOGPOGPOGPOGPOGPOGPOGPOGPO-NH₂ |
| SEQ ID NO:67 | Alexa Fluor-680-Ahx-GPOGPOGPOGPOGPOGPOGPOGPOGPO-NH₂ |
| SEQ ID NO:68 | Cyanine 3-Ahx-GPOGPOGPOGPOGPOGPOGPOGPOGPO-NH₂ |
| SEQ ID NO:69 | Cyanine 5-Ahx-GPOGPOGPOGPOGPOGPOGPOGPOGPO-NH₂ |
| SEQ ID NO:70 | Cyanine 5.5-Ahx-GPOGPOGPOGPOGPOGPOGPOGPOGPO-NH₂ |
| SEQ ID NO:71 | Cyanine 7-Ahx-GPOGPOGPOGPOGPOGPOGPOGPOGPO-NH₂ |
| SEQ ID NO:72 | Cyanine 7.5-Ahx-GPOGPOGPOGPOGPOGPOGPOGPOGPO-NH₂ |
| SEQ ID NO:73 | Cyanine 3-phycoerythrin-Ahx-GPOGPOGPOGPOGPOGPOGPOGPOGPO-NH₂ |
| SEQ ID NO:74 | Cyanine 5-phycoerythrin-Ahx-GPOGPOGPOGPOGPOGPOGPOGPOGPO-NH₂ |
| SEQ ID NO:75 | Cyanine 5.5-phycoerythrin-Ahx-GPOGPOGPOGPOGPOGPOGPOGPOGPO-NH₂ |
| SEQ ID NO:76 | Cyanine 7-phycoerythrin-Ahx-GPOGPOGPOGPOGPOGPOGPOGPOGPO-NH₂ |
| SEQ ID NO:77 | Cyanine 7.5-phycoerythrin-Ahx-GPOGPOGPOGPOGPOGPOGPOGPOGPO-NH₂ |
| SEQ ID NO:78 | Rhodamine-Ahx-GPOGPOGPOGPOGPOGPOGPOGPOGPO-NH₂ |
| SEQ ID NO:79 | Sulfo-Cyanine3-Ahx-GPOGPOGPOGPOGPOGPOGPOGPOGPO-NH₂ |
| SEQ ID NO:80 | Sulfo-Cyanine5-Ahx-GPOGPOGPOGPOGPOGPOGPOGPOGPO-NH₂ |
| SEQ ID NO:81 | Sulfo-Cyanine5.5-Ahx-GPOGPOGPOGPOGPOGPOGPOGPOGPO-NH₂ |
| SEQ ID NO:82 | Sulfo-Cyanine7-Ahx-GPOGPOGPOGPOGPOGPOGPOGPOGPO-NH₂ |
| SEQ ID NO:83 | Sulfo-Cyanine7.5-Ahx-GPOGPOGPOGPOGPOGPOGPOGPOGPO-NH₂ |
| SEQ ID NO:84 | Texas Red-Ahx-GPOGPOGPOGPOGPOGPOGPOGPOGPO-NH₂ |
| SEQ ID NO:85 | Biotin-Ahx-GfOGfOGfOGfOGfOGfOGfOGfOGfO-NH₂ |
| SEQ ID NO:86 | 5(6)-Carboxyfluorescein-Ahx-GfOGfOGfOGfOGfOGfOGfOGfOGfO-NH₂ |
| SEQ ID NO:87 | Alexa Fluor-350-Ahx-GfOGfOGfOGfOGfOGfOGfOGfOGfO-NH₂ |
| SEQ ID NO:88 | Alexa Fluor-430-Ahx-GfOGfOGfOGfOGfOGfOGfOGfOGfO-NH₂ |
| SEQ ID NO:89 | Alexa Fluor-488-Ahx-GfOGfOGfOGfOGfOGfOGfOGfOGfO-NH₂ |
| SEQ ID NO:90 | Alexa Fluor-546-Ahx-GfOGfOGfOGfOGfOGfOGfOGfOGfO-NH₂ |
| SEQ ID NO:91 | Alexa Fluor-568-Ahx-GfOGfOGfOGfOGfOGfOGfOGfOGfO-NH₂ |
| SEQ ID NO:92 | Alexa Fluor-594-Ahx-GfOGfOGfOGfOGfOGfOGfOGfOGfO-NH₂ |
| SEQ ID NO:93 | Alexa Fluor-633-Ahx-GfOGfOGfOGfOGfOGfOGfOGfOGfO-NH₂ |
| SEQ ID NO:94 | Alexa Fluor-660-Ahx-GfOGfOGfOGfOGfOGfOGfOGfOGfO-NH₂ |
| SEQ ID NO:95 | Alexa Fluor-680-Ahx-GfOGfOGfOGfOGfOGfOGfOGfOGfO-NH₂ |
| SEQ ID NO:96 | Cyanine 3-Ahx-GfOGfOGfOGfOGfOGfOGfOGfOGfO-NH₂ |
| SEQ ID NO:97 | Cyanine 5-Ahx-GfOGfOGfOGfOGfOGfOGfOGfOGfO-NH₂ |
| SEQ ID NO:98 | Cyanine 5.5-Ahx-GfOGfOGfOGfOGfOGfOGfOGfOGfO-NH₂ |
| SEQ ID NO:99 | Cyanine 7-Ahx-GfOGfOGfOGfOGfOGfOGfOGfOGfO-NH₂ |
| SEQ ID NO:100 | Cyanine 7.5-Ahx-GfOGfOGfOGfOGfOGfOGfOGfOGfO-NH₂ |
| SEQ ID NO:101 | Cyanine 3-phycoerythrin-Ahx-GfOGfOGfOGfOGfOGfOGfOGfOGfO-NH₂ |
| SEQ ID NO:102 | Cyanine 5-phycoerythrin-Ahx-GfOGfOGfOGfOGfOGfOGfOGfOGfO-NH₂ |
| SEQ ID NO:103 | Cyanine 5.5-phycoerythrin-Ahx-GfOGfOGfOGfOGfOGfOGfOGfOGfO-NH₂ |
| SEQ ID NO:104 | Cyanine 7-phycoerythrin-Ahx-GfOGfOGfOGfOGfOGfOGfOGfOGfO-NH₂ |
| SEQ ID NO:105 | Cyanine 7.5-phycoerythrin-Ahx-GfOGfOGfOGfOGfOGfOGfOGfOGfO-NH₂ |
| SEQ ID NO:106 | Rhodamine-Ahx-GfOGfOGfOGfOGfOGfOGfOGfOGfO-NH₂ |
| SEQ ID NO:107 | Sulfo-Cyanine3-Ahx-GfOGfOGfOGfOGfOGfOGfOGfOGfO-NH₂ |
| SEQ ID NO:108 | Sulfo-Cyanine5-Ahx-GfOGfOGfOGfOGfOGfOGfOGfOGfO-NH₂ |
| SEQ ID NO:109 | Sulfo-Cyanine5.5-Ahx-GfOGfOGfOGfOGfOGfOGfOGfOGfO-NH₂ |
| SEQ ID NO:110 | Sulfo-Cyanine7-Ahx-GfOGfOGfOGfOGfOGfOGfOGfOGfO-NH₂ |
| SEQ ID NO:111 | Sulfo-Cyanine7.5-Ahx-GfOGfOGfOGfOGfOGfOGfOGfOGfO-NH₂ |
| SEQ ID NO:112 | Texas Red-Ahx-GfOGfOGfOGfOGfOGfOGfOGfOGfO-NH₂ |
| SEQ ID NO:113 | Biotin-Ahx-GcOGcOGcOGcOGcOGcOGcOGcOGcO-NH₂ |
| SEQ ID NO:114 | 5(6)-Carboxyfluorescein-Ahx-GcOGcOGcOGcOGcOGcOGcOGcOGcO-NH₂ |
| SEQ ID NO:115 | Alexa Fluor-350-Ahx-GcOGcOGcOGcOGcOGcOGcOGcOGcO-NH₂ |
| SEQ ID NO:116 | Alexa Fluor-430-Ahx-GcOGcOGcOGcOGcOGcOGcOGcOGcO-NH₂ |
| SEQ ID NO:117 | Alexa Fluor-488-Ahx-GcOGcOGcOGcOGcOGcOGcOGcOGcO-NH₂ |
| SEQ ID NO:118 | Alexa Fluor-546-Ahx-GcOGcOGcOGcOGcOGcOGcOGcOGcO-NH₂ |
| SEQ ID NO:119 | Alexa Fluor-568-Ahx-GcOGcOGcOGcOGcOGcOGcOGcOGcO-NH₂ |
| SEQ ID NO:120 | Alexa Fluor-594-Ahx-GcOGcOGcOGcOGcOGcOGcOGcOGcO-NH₂ |
| SEQ ID NO:121 | Alexa Fluor-633-Ahx-GcOGcOGcOGcOGcOGcOGcOGcOGcO-NH₂ |
| SEQ ID NO:122 | Alexa Fluor-660-Ahx-GcOGcOGcOGcOGcOGcOGcOGcOGcO-NH₂ |
| SEQ ID NO:123 | Alexa Fluor-680-Ahx-GcOGcOGcOGcOGcOGcOGcOGcOGcO-NH₂ |
| SEQ ID NO:124 | Cyanine 3-Ahx-GcOGcOGcOGcOGcOGcOGcOGcOGcO-NH₂ |
| SEQ ID NO:125 | Cyanine 5-Ahx-GcOGcOGcOGcOGcOGcOGcOGcOGcO-NH₂ |
| SEQ ID NO:126 | Cyanine 5.5-Ahx-GcOGcOGcOGcOGcOGcOGcOGcOGcO-NH₂ |
| SEQ ID NO:127 | Cyanine 7-Ahx-GcOGcOGcOGcOGcOGcOGcOGcOGcO-NH₂ |
| SEQ ID NO:128 | Cyanine 7.5-Ahx-GcOGcOGcOGcOGcOGcOGcOGcOGcO-NH₂ |
| SEQ ID NO:129 | Cyanine 3-phycoerythrin-Ahx-GcOGcOGcOGcOGcOGcOGcOGcOGcO-NH₂ |
| SEQ ID NO:130 | Cyanine 5-phycoerythrin-Ahx-GcOGcOGcOGcOGcOGcOGcOGcOGcO-NH₂ |
| SEQ ID NO:131 | Cyanine 5.5-phycoerythrin-Ahx-GcOGcOGcOGcOGcOGcOGcOGcOGcO-NH₂ |
| SEQ ID NO:132 | Cyanine 7-phycoerythrin-Ahx-GcOGcOGcOGcOGcOGcOGcOGcOGcO-NH₂ |
| SEQ ID NO:134 | Cyanine 7.5-phycoerythrin-Ahx-GcOGcOGcOGcOGcOGcOGcOGcOGcO-NH₂ |
| SEQ ID NO:135 | Rhodamine-Ahx-GcOGcOGcOGcOGcOGcOGcOGcOGcO-NH₂ |
| SEQ ID NO:136 | Sulfo-Cyanine3-Ahx-GcOGcOGcOGcOGcOGcOGcOGcOGcO-NH₂ |
| SEQ ID NO:137 | Sulfo-Cyanine5-Ahx-GcOGcOGcOGcOGcOGcOGcOGcOGcO-NH₂ |
| SEQ ID NO:138 | Sulfo-Cyanine5.5-Ahx-GcOGcOGcOGcOGcOGcOGcOGcOGcO-NH₂ |
| SEQ ID NO:139 | Sulfo-Cyanine7-Ahx-GcOGcOGcOGcOGcOGcOGcOGcOGcO-NH₂ |
| SEQ ID NO:140 | Sulfo-Cyanine7.5-Ahx-GcOGcOGcOGcOGcOGcOGcOGcOGcO-NH₂ |
| SEQ ID NO:141 | Texas Red-Ahx-GcOGcOGcOGcOGcOGcOGcOGcOGcO-NH₂ |
| SEQ ID NO:142 | Biotin-GGG-GcOGcOGcOGcOGcOGcOGcOGcOGcO-NH₂ |
| SEQ ID NO:143 | 5(6)-Carboxyfluorescein-GGG-GcOGcOGcOGcOGcOGcOGcOGcOGcO-NH₂ |
| SEQ ID NO:144 | Alexa Fluor-350-GGG-GcOGcOGcOGcOGcOGcOGcOGcOGcO-NH₂ |
| SEQ ID NO:145 | Alexa Fluor-430-GGG-GcOGcOGcOGcOGcOGcOGcOGcOGcO-NH₂ |
| SEQ ID NO:146 | Alexa Fluor-488-GGG-GcOGcOGcOGcOGcOGcOGcOGcOGcO-NH₂ |
| SEQ ID NO:147 | Alexa Fluor-546-GGG-GcOGcOGcOGcOGcOGcOGcOGcOGcO-NH₂ |
| SEQ ID NO:148 | Alexa Fluor-568-GGG-GcOGcOGcOGcOGcOGcOGcOGcOGcO-NH₂ |
| SEQ ID NO:149 | Alexa Fluor-594-GGG-GcOGcOGcOGcOGcOGcOGcOGcOGcO-NH₂ |
| SEQ ID NO:150 | Alexa Fluor-633-GGG-GcOGcOGcOGcOGcOGcOGcOGcOGcO-NH₂ |
| SEQ ID NO:151 | Alexa Fluor-660-GGG-GcOGcOGcOGcOGcOGcOGcOGcOGcO-NH₂ |
| SEQ ID NO:152 | Alexa Fluor-680-GGG-GcOGcOGcOGcOGcOGcOGcOGcOGcO-NH₂ |
| SEQ ID NO:153 | Cyanine 3-GGG-GcOGcOGcOGcOGcOGcOGcOGcOGcO-NH₂ |
| SEQ ID NO:154 | Cyanine 5-GGG-GcOGcOGcOGcOGcOGcOGcOGcOGcO-NH₂ |
| SEQ ID NO:155 | Cyanine 5.5-GGG-GcOGcOGcOGcOGcOGcOGcOGcOGcO-NH₂ |
| SEQ ID NO:156 | Cyanine 7-GGG-GcOGcOGcOGcOGcOGcOGcOGcOGcO-NH₂ |
| SEQ ID NO:157 | Cyanine 7.5-GGG-GcOGcOGcOGcOGcOGcOGcOGcOGcO-NH₂ |
| SEQ ID NO:158 | Cyanine 3-phycoerythrin-GGG-GcOGcOGcOGcOGcOGcOGcOGcOGcO-NH₂ |
| SEQ ID NO:159 | Cyanine 5-phycoerythrin-GGG-GcOGcOGcOGcOGcOGcOGcOGcOGcO-NH₂ |
| SEQ ID NO:160 | Cyanine 5.5-phycoerythrin-GGG-GcOGcOGcOGcOGcOGcOGcOGcOGcO-NH₂ |
| SEQ ID NO:161 | Cyanine 7-phycoerythrin-GGG-GcOGcOGcOGcOGcOGcOGcOGcOGcO-NH₂ |
| SEQ ID NO:162 | Cyanine 7.5-phycoerythrin-GGG-GcOGcOGcOGcOGcOGcOGcOGcOGcO-NH₂ |
| SEQ ID NO:163 | Rhodamine-GGG-GcOGcOGcOGcOGcOGcOGcOGcOGcO-NH₂ |
| SEQ ID NO:164 | Sulfo-Cyanine3-GGG-GcOGcOGcOGcOGcOGcOGcOGcOGcO-NH₂ |
| SEQ ID NO:165 | Sulfo-Cyanine5-GGG-GcOGcOGcOGcOGcOGcOGcOGcOGcO-NH₂ |
| SEQ ID NO:166 | Sulfo-Cyanine5.5-GGG-GcOGcOGcOGcOGcOGcOGcOGcOGcO-NH₂ |
| SEQ ID NO:167 | Sulfo-Cyanine7-GGG-GcOGcOGcOGcOGcOGcOGcOGcOGcO-NH₂ |
| SEQ ID NO:168 | Sulfo-Cyanine7.5-GGG-GcOGcOGcOGcOGcOGcOGcOGcOGcO-NH₂ |
| SEQ ID NO:169 | Texas Red-GGG-GcOGcOGcOGcOGcOGcOGcOGcOGcO-NH₂ |
| SEQ ID NO:170 | Biotin-GGG-GOPGOPGOPGOPGOPGOPGOPGOPGOP-NH₂ |
| SEQ ID NO:171 | 5(6)-Carboxyfluorescein-GGG-GOPGOPGOPGOPGOPGOPGOPGOPGOP-NH₂ |
| SEQ ID NO:172 | Alexa Fluor-350-GGG-GOPGOPGOPGOPGOPGOPGOPGOPGOP-NH₂ |
| SEQ ID NO:173 | Alexa Fluor-430-GGG-GOPGOPGOPGOPGOPGOPGOPGOPGOP-NH₂ |
| SEQ ID NO:174 | Alexa Fluor-488-GGG-GOPGOPGOPGOPGOPGOPGOPGOPGOP-NH₂ |
| SEQ ID NO:175 | Alexa Fluor-546-GGG-GOPGOPGOPGOPGOPGOPGOPGOPGOP-NH₂ |
| SEQ ID NO:176 | Alexa Fluor-568-GGG-GOPGOPGOPGOPGOPGOPGOPGOPGOP-NH₂ |
| SEQ ID NO:177 | Alexa Fluor-594-GGG-GOPGOPGOPGOPGOPGOPGOPGOPGOP-NH₂ |
| SEQ ID NO:178 | Alexa Fluor-633-GGG-GOPGOPGOPGOPGOPGOPGOPGOPGOP-NH₂ |
| SEQ ID NO:179 | Alexa Fluor-660-GGG-GOPGOPGOPGOPGOPGOPGOPGOPGOP-NH₂ |
| SEQ ID NO:180 | Alexa Fluor-680-GGG-GOPGOPGOPGOPGOPGOPGOPGOPGOP-NH₂ |
| SEQ ID NO:181 | Cyanine 3-GGG-GOPGOPGOPGOPGOPGOPGOPGOPGOP-NH₂ |
| SEQ ID NO:182 | Cyanine 5-GGG-GOPGOPGOPGOPGOPGOPGOPGOPGOP-NH₂ |
| SEQ ID NO:183 | Cyanine 5.5-GGG-GOPGOPGOPGOPGOPGOPGOPGOPGOP-NH₂ |
| SEQ ID NO:184 | Cyanine 7-GGG-GOPGOPGOPGOPGOPGOPGOPGOPGOP-NH₂ |
| SEQ ID NO:185 | Cyanine 7.5-GGG-GOPGOPGOPGOPGOPGOPGOPGOPGOP-NH₂ |
| SEQ ID NO:186 | Cyanine 3-phycoerythrin-GGG-GOPGOPGOPGOPGOPGOPGOPGOPGOP-NH₂ |
| SEQ ID NO:187 | Cyanine 5-phycoerythrin-GGG-GOPGOPGOPGOPGOPGOPGOPGOPGOP-NH₂ |
| SEQ ID NO:188 | Cyanine 5.5-phycoerythrin-GGG-GOPGOPGOPGOPGOPGOPGOPGOPGOP-NH₂ |
| SEQ ID NO:189 | Cyanine 7-phycoerythrin-GGG-GOPGOPGOPGOPGOPGOPGOPGOPGOP-NH₂ |
| SEQ ID NO:190 | Cyanine 7.5-phycoerythrin-GGG-GOPGOPGOPGOPGOPGOPGOPGOPGOP-NH₂ |
| SEQ ID NO:191 | Rhodamine-GGG-GOPGOPGOPGOPGOPGOPGOPGOPGOP-NH₂ |
| SEQ ID NO:192 | Sulfo-Cyanine3-GGG-GOPGOPGOPGOPGOPGOPGOPGOPGOP-NH₂ |
| SEQ ID NO:193 | Sulfo-Cyanine5-GGG-GOPGOPGOPGOPGOPGOPGOPGOPGOP-NH₂ |
| SEQ ID NO:194 | Sulfo-Cyanine5.5-GGG-GOPGOPGOPGOPGOPGOPGOPGOPGOP-NH₂ |
| SEQ ID NO:195 | Sulfo-Cyanine7-GGG-GOPGOPGOPGOPGOPGOPGOPGOPGOP-NH₂ |
| SEQ ID NO:196 | Sulfo-Cyanine7.5-GGG-GOPGOPGOPGOPGOPGOPGOPGOPGOP-NH₂ |
| SEQ ID NO:197 | Texas Red-GGG-GOPGOPGOPGOPGOPGOPGOPGOPGOP-NH₂ |
| SEQ ID NO:198 | Biotin-Ahx-GOPGOPGOPGOPGOPGOPGOPGOPGOP-NH₂ |
| SEQ ID NO:199 | 5(6)-Carboxyfluorescein-Ahx-GOPGOPGOPGOPGOPGOPGOPGOPGOP-NH₂ |
| SEQ ID NO:200 | Alexa Fluor-350-Ahx-GOPGOPGOPGOPGOPGOPGOPGOPGOP-NH₂ |
| SEQ ID NO:201 | Alexa Fluor-430-Ahx-GOPGOPGOPGOPGOPGOPGOPGOPGOP-NH₂ |
| SEQ ID NO:202 | Alexa Fluor-488-Ahx-GOPGOPGOPGOPGOPGOPGOPGOPGOP-NH₂ |
| SEQ ID NO:203 | Alexa Fluor-546-Ahx-GOPGOPGOPGOPGOPGOPGOPGOPGOP-NH₂ |
| SEQ ID NO:204 | Alexa Fluor-568-Ahx-GOPGOPGOPGOPGOPGOPGOPGOPGOP-NH₂ |
| SEQ ID NO:205 | Alexa Fluor-594-Ahx-GOPGOPGOPGOPGOPGOPGOPGOPGOP-NH₂ |
| SEQ ID NO:206 | Alexa Fluor-633-Ahx-GOPGOPGOPGOPGOPGOPGOPGOPGOP-NH₂ |
| SEQ ID NO:207 | Alexa Fluor-660-Ahx-GOPGOPGOPGOPGOPGOPGOPGOPGOP-NH₂ |
| SEQ ID NO:208 | Alexa Fluor-680-Ahx-GOPGOPGOPGOPGOPGOPGOPGOPGOP-NH₂ |
| SEQ ID NO:209 | Cyanine 3-Ahx-GOPGOPGOPGOPGOPGOPGOPGOPGOP-NH₂ |
| SEQ ID NO:210 | Cyanine 5-Ahx-GOPGOPGOPGOPGOPGOPGOPGOPGOP-NH₂ |
| SEQ ID NO:211 | Cyanine 5.5-Ahx-GOPGOPGOPGOPGOPGOPGOPGOPGOP-NH₂ |
| SEQ ID NO:212 | Cyanine 7-Ahx-GOPGOPGOPGOPGOPGOPGOPGOPGOP-NH₂ |
| SEQ ID NO:213 | Cyanine 7.5-Ahx-GOPGOPGOPGOPGOPGOPGOPGOPGOP-NH₂ |
| SEQ ID NO:214 | Cyanine 3-phycoerythrin-Ahx-GOPGOPGOPGOPGOPGOPGOPGOPGOP-NH₂ |
| SEQ ID NO:215 | Cyanine 5-phycoerythrin-Ahx-GOPGOPGOPGOPGOPGOPGOPGOPGOP-NH₂ |
| SEQ ID NO:216 | Cyanine 5.5-phycoerythrin-Ahx-GOPGOPGOPGOPGOPGOPGOPGOPGOP-NH₂ |
| SEQ ID NO:217 | Cyanine 7-phycoerythrin-Ahx-GOPGOPGOPGOPGOPGOPGOPGOPGOP-NH₂ |
| SEQ ID NO:218 | Cyanine 7.5-phycoerythrin-Ahx-GOPGOPGOPGOPGOPGOPGOPGOPGOP-NH₂ |
| SEQ ID NO:219 | Rhodamine-Ahx-GOPGOPGOPGOPGOPGOPGOPGOPGOP-NH₂ |
| SEQ ID NO:220 | Sulfo-Cyanine3-Ahx-GOPGOPGOPGOPGOPGOPGOPGOPGOP-NH₂ |
| SEQ ID NO:221 | Sulfo-Cyanine5-Ahx-GOPGOPGOPGOPGOPGOPGOPGOPGOP-NH₂ |
| SEQ ID NO:222 | Sulfo-Cyanine5.5-Ahx-GOPGOPGOPGOPGOPGOPGOPGOPGOP-NH₂ |
| SEQ ID NO:223 | Sulfo-Cyanine7-Ahx-GOPGOPGOPGOPGOPGOPGOPGOPGOP-NH₂ |
| SEQ ID NO:224 | Sulfo-Cyanine7.5-Ahx-GOPGOPGOPGOPGOPGOPGOPGOPGOP-NH₂ |
| SEQ ID NO:225 | Texas Red-Ahx-GOPGOPGOPGOPGOPGOPGOPGOPGOP-NH₂ |
| SEQ ID NO:226 | Biotin-GGG-GPPGPPGPPGPPGPPGPPGPPGPPGPP-NH₂ |
| SEQ ID NO:227 | 5(6)-Carboxyfluorescein-GGG-GPPGPPGPPGPPGPPGPPGPPGPPGPP-NH₂ |
| SEQ ID NO:228 | Alexa Fluor-350-GGG-GPPGPPGPPGPPGPPGPPGPPGPPGPP-NH₂ |
| SEQ ID NO:229 | Alexa Fluor-430-GGG-GPPGPPGPPGPPGPPGPPGPPGPPGPP-NH₂ |
| SEQ ID NO:230 | Alexa Fluor-488-GGG-GPPGPPGPPGPPGPPGPPGPPGPPGPP-NH₂ |
| SEQ ID NO:231 | Alexa Fluor-546-GGG-GPPGPPGPPGPPGPPGPPGPPGPPGPP-NH₂ |
| SEQ ID NO:232 | Alexa Fluor-568-GGG-GPPGPPGPPGPPGPPGPPGPPGPPGPP-NH₂ |
| SEQ ID NO:233 | Alexa Fluor-594-GGG-GPPGPPGPPGPPGPPGPPGPPGPPGPP-NH₂ |
| SEQ ID NO:234 | Alexa Fluor-633-GGG-GPPGPPGPPGPPGPPGPPGPPGPPGPP-NH₂ |
| SEQ ID NO:235 | Alexa Fluor-660-GGG-GPPGPPGPPGPPGPPGPPGPPGPPGPP-NH₂ |
| SEQ ID NO:236 | Alexa Fluor-680-GGG-GPPGPPGPPGPPGPPGPPGPPGPPGPP-NH₂ |
| SEQ ID NO:237 | Cyanine 3-GGG-GPPGPPGPPGPPGPPGPPGPPGPPGPP-NH₂ |
| SEQ ID NO:238 | Cyanine 5-GGG-GPPGPPGPPGPPGPPGPPGPPGPPGPP-NH₂ |
| SEQ ID NO:239 | Cyanine 5.5-GGG-GPPGPPGPPGPPGPPGPPGPPGPPGPP-NH₂ |
| SEQ ID NO:240 | Cyanine 7-GGG-GPPGPPGPPGPPGPPGPPGPPGPPGPP-NH₂ |
| SEQ ID NO:241 | Cyanine 7.5-GGG-GPPGPPGPPGPPGPPGPPGPPGPPGPP-NH₂ |
| SEQ ID NO:242 | Cyanine 3-phycoerythrin-GGG-GPPGPPGPPGPPGPPGPPGPPGPPGPP-NH₂ |
| SEQ ID NO:243 | Cyanine 5-phycoerythrin-GGG-GPPGPPGPPGPPGPPGPPGPPGPPGPP-NH₂ |
| SEQ ID NO:244 | Cyanine 5.5-phycoerythrin-GGG-GPPGPPGPPGPPGPPGPPGPPGPPGPP-NH₂ |
| SEQ ID NO:245 | Cyanine 7-phycoerythrin-GGG-GPPGPPGPPGPPGPPGPPGPPGPPGPP-NH₂ |
| SEQ ID NO:246 | Cyanine 7.5-phycoerythrin-GGG-GPPGPPGPPGPPGPPGPPGPPGPPGPP-NH₂ |
| SEQ ID NO:247 | Rhodamine-GGG-GPPGPPGPPGPPGPPGPPGPPGPPGPP-NH₂ |
| SEQ ID NO:248 | Sulfo-Cyanine3-GGG-GPPGPPGPPGPPGPPGPPGPPGPPGPP-NH₂ |
| SEQ ID NO:249 | Sulfo-Cyanine5-GGG-GPPGPPGPPGPPGPPGPPGPPGPPGPP-NH₂ |
| SEQ ID NO:250 | Sulfo-Cyanine5.5-GGG-GPPGPPGPPGPPGPPGPPGPPGPPGPP-NH₂ |
| SEQ ID NO:251 | Sulfo-Cyanine7-GGG-GPPGPPGPPGPPGPPGPPGPPGPPGPP-NH₂ |
| SEQ ID NO:252 | Sulfo-Cyanine7.5-GGG-GPPGPPGPPGPPGPPGPPGPPGPPGPP-NH₂ |
| SEQ ID NO:253 | Texas Red-GGG-GPPGPPGPPGPPGPPGPPGPPGPPGPP-NH₂ |
| SEQ ID NO:254 | Biotin-Ahx-GPPGPPGPPGPPGPPGPPGPPGPPGPP-NH₂ |
| SEQ ID NO:255 | 5(6)-Carboxyfluorescein-Ahx-GPPGPPGPPGPPGPPGPPGPPGPPGPP-NH₂ |
| SEQ ID NO:256 | Alexa Fluor-350-Ahx-GPPGPPGPPGPPGPPGPPGPPGPPGPP-NH₂ |
| SEQ ID NO:257 | Alexa Fluor-430-Ahx-GPPGPPGPPGPPGPPGPPGPPGPPGPP-NH₂ |
| SEQ ID NO:258 | Alexa Fluor-488-Ahx-GPPGPPGPPGPPGPPGPPGPPGPPGPP-NH₂ |
| SEQ ID NO:259 | Alexa Fluor-546-Ahx-GPPGPPGPPGPPGPPGPPGPPGPPGPP-NH₂ |
| SEQ ID NO:260 | Alexa Fluor-568-Ahx-GPPGPPGPPGPPGPPGPPGPPGPPGPP-NH₂ |
| SEQ ID NO:261 | Alexa Fluor-594-Ahx-GPPGPPGPPGPPGPPGPPGPPGPPGPP-NH₂ |
| SEQ ID NO:262 | Alexa Fluor-633-Ahx-GPPGPPGPPGPPGPPGPPGPPGPPGPP-NH₂ |
| SEQ ID NO:263 | Alexa Fluor-660-Ahx-GPPGPPGPPGPPGPPGPPGPPGPPGPP-NH₂ |
| SEQ ID NO:264 | Alexa Fluor-680-Ahx-GPPGPPGPPGPPGPPGPPGPPGPPGPP-NH₂ |
| SEQ ID NO:265 | Cyanine 3-Ahx-GPPGPPGPPGPPGPPGPPGPPGPPGPP-NH₂ |
| SEQ ID NO:266 | Cyanine 5-Ahx-GPPGPPGPPGPPGPPGPPGPPGPPGPP-NH₂ |
| SEQ ID NO:267 | Cyanine 5.5-Ahx-GPPGPPGPPGPPGPPGPPGPPGPPGPP-NH₂ |
| SEQ ID NO:268 | Cyanine 7-Ahx-GPPGPPGPPGPPGPPGPPGPPGPPGPP-NH₂ |
| SEQ ID NO:269 | Cyanine 7.5-Ahx-GPPGPPGPPGPPGPPGPPGPPGPPGPP-NH₂ |
| SEQ ID NO:270 | Cyanine 3-phycoerythrin-Ahx-GPPGPPGPPGPPGPPGPPGPPGPPGPP-NH₂ |
| SEQ ID NO:271 | Cyanine 5-phycoerythrin-Ahx-GPPGPPGPPGPPGPPGPPGPPGPPGPP-NH₂ |
| SEQ ID NO:272 | Cyanine 5.5-phycoerythrin-Ahx-GPPGPPGPPGPPGPPGPPGPPGPPGPP-NH₂ |
| SEQ ID NO:273 | Cyanine 7-phycoerythrin-Ahx-GPPGPPGPPGPPGPPGPPGPPGPPGPP-NH₂ |
| SEQ ID NO:274 | Cyanine 7.5-phycoerythrin-Ahx-GPPGPPGPPGPPGPPGPPGPPGPPGPP-NH₂ |
| SEQ ID NO:275 | Rhodamine-Ahx-GPPGPPGPPGPPGPPGPPGPPGPPGPP-NH₂ |
| SEQ ID NO:276 | Sulfo-Cyanine3-Ahx-GPPGPPGPPGPPGPPGPPGPPGPPGPP-NH₂ |
| SEQ ID NO:277 | Sulfo-Cyanine5-Ahx-GPPGPPGPPGPPGPPGPPGPPGPPGPP-NH₂ |
| SEQ ID NO:278 | Sulfo-Cyanine5.5-Ahx-GPPGPPGPPGPPGPPGPPGPPGPPGPP-NH₂ |
| SEQ ID NO:279 | Sulfo-Cyanine7-Ahx-GPPGPPGPPGPPGPPGPPGPPGPPGPP-NH₂ |
| SEQ ID NO:280 | Sulfo-Cyanine7.5-Ahx-GPPGPPGPPGPPGPPGPPGPPGPPGPP-NH₂ |
| SEQ ID NO:281 | Texas Red-Ahx-GPPGPPGPPGPPGPPGPPGPPGPPGPP-NH₂ |
| SEQ ID NO:282 | Biotin-GGG-GPFGPFGPFGPFGPFGPFGPFGPFGPF-NH₂ |
| SEQ ID NO:283 | 5(6)-Carboxyfluorescein-GGG-GPFGPFGPFGPFGPFGPFGPFGPFGPF-NH₂ |
| SEQ ID NO:284 | Alexa Fluor-350-GGG-GPFGPFGPFGPFGPFGPFGPFGPFGPF-NH₂ |
| SEQ ID NO:285 | Alexa Fluor-430-GGG-GPFGPFGPFGPFGPFGPFGPFGPFGPF-NH₂ |
| SEQ ID NO:286 | Alexa Fluor-488-GGG-GPFGPFGPFGPFGPFGPFGPFGPFGPF-NH₂ |
| SEQ ID NO:287 | Alexa Fluor-546-GGG-GPFGPFGPFGPFGPFGPFGPFGPFGPF-NH₂ |
| SEQ ID NO:288 | Alexa Fluor-568-GGG-GPFGPFGPFGPFGPFGPFGPFGPFGPF-NH₂ |
| SEQ ID NO:289 | Alexa Fluor-594-GGG-GPFGPFGPFGPFGPFGPFGPFGPFGPF-NH₂ |
| SEQ ID NO:290 | Alexa Fluor-633-GGG-GPFGPFGPFGPFGPFGPFGPFGPFGPF-NH₂ |
| SEQ ID NO:291 | Alexa Fluor-660-GGG-GPFGPFGPFGPFGPFGPFGPFGPFGPF-NH₂ |
| SEQ ID NO:292 | Alexa Fluor-680-GGG-GPFGPFGPFGPFGPFGPFGPFGPFGPF-NH₂ |
| SEQ ID NO:293 | Cyanine 3-GGG-GPFGPFGPFGPFGPFGPFGPFGPFGPF-NH₂ |
| SEQ ID NO:294 | Cyanine 5-GGG-GPFGPFGPFGPFGPFGPFGPFGPFGPF-NH₂ |
| SEQ ID NO:295 | Cyanine 5.5-GGG-GPFGPFGPFGPFGPFGPFGPFGPFGPF-NH₂ |
| SEQ ID NO:296 | Cyanine 7-GGG-GPFGPFGPFGPFGPFGPFGPFGPFGPF-NH₂ |
| SEQ ID NO:297 | Cyanine 7.5-GGG-GPFGPFGPFGPFGPFGPFGPFGPFGPF-NH₂ |
| SEQ ID NO:298 | Cyanine 3-phycoerythrin-GGG-GPFGPFGPFGPFGPFGPFGPFGPFGPF-NH₂ |
| SEQ ID NO:299 | Cyanine 5-phycoerythrin-GGG-GPFGPFGPFGPFGPFGPFGPFGPFGPF-NH₂ |
| SEQ ID NO:300 | Cyanine 5.5-phycoerythrin-GGG-GPFGPFGPFGPFGPFGPFGPFGPFGPF-NH₂ |
| SEQ ID NO:301 | Cyanine 7-phycoerythrin-GGG-GPFGPFGPFGPFGPFGPFGPFGPFGPF-NH₂ |
| SEQ ID NO:302 | Cyanine 7.5-phycoerythrin-GGG-GPFGPFGPFGPFGPFGPFGPFGPFGPF-NH₂ |
| SEQ ID NO:303 | Rhodamine-GGG-GPFGPFGPFGPFGPFGPFGPFGPFGPF-NH₂ |
| SEQ ID NO:304 | Sulfo-Cyanine3-GGG-GPFGPFGPFGPFGPFGPFGPFGPFGPF-NH₂ |
| SEQ ID NO:305 | Sulfo-Cyanine5-GGG-GPFGPFGPFGPFGPFGPFGPFGPFGPF-NH₂ |
| SEQ ID NO:306 | Sulfo-Cyanine5.5-GGG-GPFGPFGPFGPFGPFGPFGPFGPFGPF-NH₂ |
| SEQ ID NO:307 | Sulfo-Cyanine7-GGG-GPFGPFGPFGPFGPFGPFGPFGPFGPF-NH₂ |
| SEQ ID NO:308 | Sulfo-Cyanine7.5-GGG-GPFGPFGPFGPFGPFGPFGPFGPFGPF-NH₂ |
| SEQ ID NO:309 | Texas Red-GGG-GPFGPFGPFGPFGPFGPFGPFGPFGPF-NH₂ |
| SEQ ID NO:310 | Biotin-Ahx-GPFGPFGPFGPFGPFGPFGPFGPFGPF-NH₂ |
| SEQ ID NO:311 | 5(6)-Carboxyfluorescein-Ahx-GPFGPFGPFGPFGPFGPFGPFGPFGPF-NH₂ |
| SEQ ID NO:312 | Alexa Fluor-350-Ahx-GPFGPFGPFGPFGPFGPFGPFGPFGPF-NH₂ |
| SEQ ID NO:313 | Alexa Fluor-430-Ahx-GPFGPFGPFGPFGPFGPFGPFGPFGPF-NH₂ |
| SEQ ID NO:314 | Alexa Fluor-488-Ahx-GPFGPFGPFGPFGPFGPFGPFGPFGPF-NH₂ |
| SEQ ID NO:315 | Alexa Fluor-546-Ahx-GPFGPFGPFGPFGPFGPFGPFGPFGPF-NH₂ |
| SEQ ID NO:316 | Alexa Fluor-568-Ahx-GPFGPFGPFGPFGPFGPFGPFGPFGPF-NH₂ |
| SEQ ID NO:317 | Alexa Fluor-594-Ahx-GPFGPFGPFGPFGPFGPFGPFGPFGPF-NH₂ |
| SEQ ID NO:318 | Alexa Fluor-633-Ahx-GPFGPFGPFGPFGPFGPFGPFGPFGPF-NH₂ |
| SEQ ID NO:319 | Alexa Fluor-660-Ahx-GPFGPFGPFGPFGPFGPFGPFGPFGPF-NH₂ |
| SEQ ID NO:320 | Alexa Fluor-680-Ahx-GPFGPFGPFGPFGPFGPFGPFGPFGPF-NH₂ |
| SEQ ID NO:321 | Cyanine 3-Ahx-GPFGPFGPFGPFGPFGPFGPFGPFGPF-NH₂ |
| SEQ ID NO:322 | Cyanine 5-Ahx-GPFGPFGPFGPFGPFGPFGPFGPFGPF-NH₂ |
| SEQ ID NO:323 | Cyanine 5.5-Ahx-GPFGPFGPFGPFGPFGPFGPFGPFGPF-NH₂ |
| SEQ ID NO:324 | Cyanine 7-Ahx-GPFGPFGPFGPFGPFGPFGPFGPFGPF-NH₂ |
| SEQ ID NO:325 | Cyanine 7.5-Ahx-GPFGPFGPFGPFGPFGPFGPFGPFGPF-NH₂ |
| SEQ ID NO:326 | Cyanine 3-phycoerythrin-Ahx-GPFGPFGPFGPFGPFGPFGPFGPFGPF-NH₂ |
| SEQ ID NO:327 | Cyanine 5-phycoerythrin-Ahx-GPFGPFGPFGPFGPFGPFGPFGPFGPF-NH₂ |
| SEQ ID NO:328 | Cyanine 5.5-phycoerythrin-Ahx-GPFGPFGPFGPFGPFGPFGPFGPFGPF-NH₂ |
| SEQ ID NO:329 | Cyanine 7-phycoerythrin-Ahx-GPFGPFGPFGPFGPFGPFGPFGPFGPF-NH₂ |
| SEQ ID NO:330 | Cyanine 7.5-phycoerythrin-Ahx-GPFGPFGPFGPFGPFGPFGPFGPFGPF-NH₂ |
| SEQ ID NO:331 | Rhodamine-Ahx-GPFGPFGPFGPFGPFGPFGPFGPFGPF-NH₂ |
| SEQ ID NO:332 | Sulfo-Cyanine3-Ahx-GPFGPFGPFGPFGPFGPFGPFGPFGPF-NH₂ |
| SEQ ID NO:333 | Sulfo-Cyanine5-Ahx-GPFGPFGPFGPFGPFGPFGPFGPFGPF-NH₂ |
| SEQ ID NO:334 | Sulfo-Cyanine5.5-Ahx-GPFGPFGPFGPFGPFGPFGPFGPFGPF-NH₂ |
| SEQ ID NO:335 | Sulfo-Cyanine7-Ahx-GPFGPFGPFGPFGPFGPFGPFGPFGPF-NH₂ |
| SEQ ID NO:336 | Sulfo-Cyanine7.5-Ahx-GPFGPFGPFGPFGPFGPFGPFGPFGPF-NH₂ |
| SEQ ID NO:337 | Texas Red-Ahx-GPFGPFGPFGPFGPFGPFGPFGPFGPF-NH₂ |

In certain sequences provided in Table 1 above, 'GGG' represents a Triple Glycine linker. In certain sequences provided in Table 1 above, 'NH₂' represents an amidated C-terminus. In certain sequences provided in Table 1 above, the 'f' in a 'GfO' sequence represents a 2S, 4S-4-fluoroproline (cis conformation). In certain sequences provided in Table 1 above, 'Ahx' represents a 6-aminohexanoic acid linker. In certain sequences provided in Table 1 above, the "c" in a 'GcO' sequences represents a 2S, 4S-4-chloroproline (cis confirmation). In certain sequences provided in Table 1 above, the 'F' in a 'GPF' sequence represents a 2S, 4R-4-fluoroproline (trans conformation).

In some embodiments, a method of the present disclosure can comprise contacting a sample with a CHP having a sequence selected from SEQ ID NO.: 1 - 337 listed in Table 1 above without containing a GGG or AHX spacer molecule. In some embodiments, the sequence comprises a CHP of Formula 1, L-Sₘ-(Gly-X-Y)₃₋₂₀, where S is a spacer molecule and m is 0.

In some embodiments, a method of the present disclosure can comprise contacting a sample with a CHP, wherein the CHP sequence comprises a (Gly-X-Y)₃₋₂₀ repeating portion, and wherein the repeating portion of the CHP has a sequence selected from SEQ ID NO.: 338-349 (see, e.g., Table 2).

**Table 2 -Sequences for Repeating Portion of Collagen Hybridizing Peptides**

| **Identifier** | **Sequence (One-Letter Amino Acid Symbols)** |
|---|---|
| SEQ ID NO:338 | (GPO)₃₋₂₀, where G = glycine, P = proline, O = hydroxyproline |
| SEQ ID NO:339 | (GfO)₃₋₂₀ where G = glycine, f = cis-fluoroproline (2S,4S-4-fluoroproline), O = hydroxyproline |
| SEQ ID NO:340 | (GOP)₃₋₂₀ where G = glycine, P = proline, O = hydroxyproline |
| SEQ ID NO:341 | (_{az}GPO)₃₋₂₀ where _{az}G = aza-Glycine, P = proline, O = hydroxyproline |
| SEQ ID NO:342 | (_{az}GfO)₃₋₂₀ where _{az}G = aza-Glycine, f = cis-fluoroproline (2S,4S-4-fluoroproline), O = Hydroxyproline |
| SEQ ID NO:343 | (GPF)₃₋₂₀ where G = Glycine, P = Proline, F = trans-fluoroproline (2S,4R-4-fluoroproline) |
| SEQ ID NO: 344 | (GPP)₃₋₂₀, where G = glycine, P = proline, P = proline |
| SEQ ID NO: 345 | (_{az}GPP)₃₋₂₀, where _{az}G = aza-glycine, P = proline, P = proline |
| SEQ ID NO: 346 | (_{az}GcO)₃₋₂₀, where _{az}G = aza-glycine, c = cis-chloroproline (2S,4S-4-chloroproline), O = trans-hydroxyproline (2S, 4R-hydroxyproline) |
| SEQ ID NO: 347 | (GcO)₃₋₂₀, where G = glycine, c = cis-chloroproline (2S,4S-4-chloroproline), O = trans-hydroxyproline (2S, 4R-hydroxyproline) |
| SEQ ID NO:348 | (_{az}GOP)₃₋₂₀ where _{az}G = aza-Glycine, P = proline, O = hydroxyproline |
| SEQ ID NO:349 | (_{az}GPF)₃₋₂₀ where _{az}G = aza-Glycine, P = Proline, F = trans-fluoroproline (2S,4R-4-fluoroproline) |

Other Embodiments and Equivalents including a dimeric version of each sequence listed in Tables 1 and 2. In certain embodiments, a dimeric sequence can differ from an amino acid sequence as provided in any of Tables 1 and 2 by 1 amino acid, 2 amino acids, 3 amino acids, 4 amino acids 5 amino acids, 6 amino acids, 7 amino acids, 8 amino acids, 9 amino acids, 10 amino acids or greater than 10 amino acids. In certain embodiments, a dimeric sequence can comprise a glycine offset and/or a lysine branch point.

The words "right", "left", "lower" and "upper" designate directions in the drawings to which reference is made. Unless specifically set forth herein, the terms "a", "an" and "the" are not limited to one element but instead should be read as meaning "at least one".

Ranges recited herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

### EXAMPLES

### Example 1 - Determining the relative content of degraded collagen in a tissue using CHPs

Two neighboring sections of the same diseased mouse heart tissue, one treated with thorough heat denaturation (*e.g*., 10 min incubation in 90 °C water bath for frozen sections, or two rounds of 5 min heat-mediated antigen retrieval at 95 °C) and the other not treated at all, are stained with the 5-FAM- or biotin-dimeric CHP (diCHP) under the same conditions. The diCHP signal from the fully denatured section represents the total collagen content, whereas the signal in the untreated section comes from the naturally degraded collagen in the disease condition. The ratio between the fluorescence signals detected at the same region of interest within the two slides provides an estimation of what percentage of collagen is degraded in the area.

The fluorescence signals can be quantified using an ImageJ-based protocol. The contents of degraded collagen for the hearts harvested at the early and late stages post MI (1 week vs. 3 weeks) are compared with normal hearts as negative controls. For each group at each time point, 5 hearts are analyzed; for each heart, 10 frozen sections and 10 paraffin sections are stained to allow statistical analysis. To confirm the accuracy of this assay, the level of the degraded collagen of the MI heart tissues using frozen sections and a trypsin-hydroxyproline assay are quantified. The sections are treated with a trypsin solution which selectively solubilizes the degraded, unfolded collagen; the collagen contents within the trypsin solution and the remaining tissue section are quantified by the standard hydroxyproline assay, and the percentage of the protease-degraded collagen originally present in the tissue can be calculated. Because the trypsin assay only works on unfixed cryosections and only measure the average percentage over the whole section, the results of the trypsin assay are compared to the percentage quantified from the whole section scans of the frozen slides stained with di-CHP.

### Example 2 - Determining total collagen content using CHPs as compared to other staining techniques

A direct stain comparison study was conducted using PSR, MT, Herovici's stain, and collagen I and collagen III antibodies in order to detect total collagen in a fibrotic mouse liver model to compare them with the biotin labeled CHPs (B-CHPs). All staining and image analysis was performed by HistoTox Labs, a third-party contract research organization, in order to prevent bias in evaluation and ensure all stains were carried out correctly. The severity of fibrosis in each slide was scored by a veterinary pathologist.

FIGS. 5A - 5J show representative photomicrographs of mouse livers stained using five different methods. Serial sections were taken from a healthy (control) mouse liver (FIGS. 5A - 5E) and a fibrotic mouse liver 8 weeks after injection with CCl₄ (FIGS. 5F - 5J). Collagen, identified by arrows in all photos, is stained blue in MT, pink/red in PSR, pink/red for mature collagen and blue for young collagen in Herovici's, dark to light brown for Col I/III cocktail, and dark brown in CHP staining. Other features of interest are labeled as: C-central vein, H-hepatocytes, P-portal triads. Magnification was 40X, scale bar = 200 µM.

As evident from the photos in FIGS 5A - 5J B-CHP performed just as well, if not better, than all other stains. Comparing the spatial staining pattern of CHP and all other stains, including antibody Col I/III cocktail, CHPs allowed easier visualization of collagen and pathologists were able to easily identify collagen without needing to interpret shades of color. Moreover, B-CHP was the only stain that identified reticulin fibers, indicating CHPs may also be used in place of silver stains for reticular fibers (e.g., Gomori's reticulin stain, Gordon and Sweet's reticulin stain, or Movat Pentachrome stain). The reticulin stain is most useful for identifying changes to the hepatic architecture (loss of hepatocytes, thickening of hepatic cords, changes in lobulation, fibrosis/cirrhosis etc.).

In addition to standard histology assessment, each of the slides was assessed using automated image analysis to quantify the area of collagen stained in each sample. For all images, regions of interest were generated to include liver tissue, but exclude artifacts (folds, tears, etc.) large blood vessels, and non-liver tissue. Regions of interest were then subjected to several imaging filters to separate positively stained areas from negative areas. The positive area was quantified, then compared to the total area in the region of interest.

In PSR staining, collagen appears bright red and remaining tissue a pale yellow. These images were easily characterized using automated image analysis without issue due to the high contrast between positive and negative regions. In Masson's Trichrome, collagen appears blue, and cells appear red. However, some endothelial cells exhibited weak blue staining in their cytoplasm and as a result, were intermittently detected by the automated image analysis even after careful thresholding. This likely resulted in an over-detection of collagen during quantification. Herovici-stained sections exhibited pink/red staining for mature collagen and pale blue for young collagen. Hepatocyte cytoplasm was stained a pale pink to pink-purple and mineral regions were stained dark purple to black. Overall, contrast was very-poor for Herovici-stained tissue, and as a result, the image analysis could only be performed for the mature collagen (pink/red) as the young collagen could not be differentiated from the negatively stained region.

CHP-stained sections exhibited light brown staining of collagen fibers (DAB staining of B-CHP). Negative regions were stained light blue to grey for cytoplasm and dark blue for nuclei (due to hematoxylin counterstain). Image analysis was generally specific to CHP-labeled collagen fibrils, but some areas of elevated background staining required the algorithm to slightly under-detect CHP-stained regions.

FIG. 6 shows collagen quantification by image analysis. Collagen was detected in all samples, with low overall collagen detected in naïve samples, and significantly higher quantities of collagen detected in diseased samples. In naïve samples the total collagen was low across all samples and staining methods. However, substantial differences were observed between stains in samples from diseased tissue. The most collagen was detected using Masson's Trichrome. However, as noted above, the weak blue staining of hepatocyte cytoplasm interfered with specific analysis, and the quantity of collagen is likely overrepresented.

PSR and CHP with HIER were highly similar and likely represent an accurate assessment of total collagen content. Herovici's staining was difficult to accurately assess using image analysis and had the smallest area of detected collagen. In addition, consistent with CHP's specificity for denatured collagen vs all collagen, tissue sections that did not undergo HIER had a lower amount of collagen detected, indicating that both intact and denatured collagen are elevated in the fibrotic tissue. Representative images and analysis from fibrotic samples are shown in FIG. 7.

### Example 3 - Detecting total and damaged collagen in human liver biopsies

Experiments were performed to assess (i) whether CHPs could be used as a reliable label to replace existing histochemical approaches and collagen proportionate area in the assessment of chronic liver injury, and (ii) whether CHPs can they provide additional clinically important information that might predict liver related outcome.

### Methods

Percutaneous biopsies from 76 well characterized patients with NAFLD were used as was a series of explant tissues including those with bridging necrosis and both inactive and active cirrhosis. Sections were incubated with biotinylated CHPs at room temperature to detect damaged collagen; sections that had been pre-heated at 80 °C were used to analyze total collagen content. The intensity and area of labelling was assessed (i) using a semi quantitative index and (ii) in scanned slides using QuPath; ratios of damaged: total collagen were calculated. Correlations were sought with clinical parameters and with grade and stage of disease (NIH CRN scoring system).

### Results and discussion

CHPs provide a reliable tool for the detection of damaged and total collagen in routinely processed human liver biopsies and for the latter may be more consistent than conventional histochemical approaches. In NAFLD, the ratio of damaged to total collagen significantly correlates with CRN stage of disease (stage 1 v stage 4 p<0.00001). Advantageously, the amount of damaged remodeled collagen is significantly lower in those considered to be slow/medium progressors compared with fast progressors (p=0.023), suggesting that these may be of high value in prognostication and in clinical trials.

FIG. 8 shows representative photographs of B-CHP treated explant tissues obtained from subjects across all stages of NAFLD (with stage 2 and stage 4 NAFLD shown). The modified H-score for the slow/medium progressors was 15.16 (mean), and for fast progressors was 29.29 (mean) with a t-value of -2.3397 and a p-value of 0.023. Thus, CHP staining for remodeling collagen is useful for prognosis of NAFLD progression.

FIG. 9 shows representative photographs showing total and damaged collagen in B-CHP treated explant tissues obtained from subjects across all stages of NAFLD (withstage 1, stage 2 and stage 4 NAFLD shown). The **T**otal to **D**amaged **R**atios (TDR) determined from the images are as follows: stage 1 - 17.14 (mean); stage 2 - 8.3 (mean); and stage 4 - 11.12 (mean). Comparison of the TDRs between stage 1 and stage 4 (p <0.00001), and between stage 2 and stage 4 (p=0.237) and between stage 3 and stage 4 (not shown) (p=0.0197) indicate that the TDRs provide a statistically significant marker between different stages of the disease. TDR is calculated using HIER mediated CHP staining and non HIER mediated CHP staining.

Further, exploration of CHP probes in other chronic fibrotic conditions could provide similar diagnostic/prognostic insights.

### Example 4 - Determining total collagen content in formalin-fixed paraffin-embedded (FFPE) tissue section using ImageJ/FIJI

### Total Collagen Content

To use CHPs for evaluating the total collagen content within a FFPE tissue section, the collagen must be fully denatured to allow for CHP binding on all available collagen. After deparaffinization, the tissue sections are heated to thermally denature the collagen. The tests indicate that the long heating periods used in heat induced epitope retrieval (HIER) methods are sufficient to completely denature the collagen in the sample (regardless of the buffer used). The tissue sections shown herein were placed in 50 mL of citrate buffer in a tissue steamer for 45 minutes at 95-100 °C. Alternatively, to denature the collagen content in a frozen tissue slide, a water bath can be used to heat up a sealed 50 mL tube containing DI water to temperatures over 85 °C. After heating, the hot DI water can be pipetted onto the tissue samples and allowed to sit for 5 minutes (repeat 10X).

### Experimental Protocol

FFPE Sections: perform deparaffinization prior to CHP staining by submerging sections for 2 x 5-minute washes in xylenes, 100% ethanol, 95% ethanol, 50% ethanol, and DI water in this order.

Perform HIER or purposefully heat denature the tissue for the determination of total collagen content.

Create a solution for CHP staining by dissolving CHP powder in 1× PBS so that the concentration is within a 5-20 µM range. The exact concentration depends on the optimized parameters for the tissue section and the volume needed.

Heat CHP solution to 80 °C. Since CHP can self-assemble into homotrimers in solution over time (e.g., during storage at 4 °C) and lose its driving force for collagen hybridization, the trimeric CHP needs to be thermally dissociated to single strands by heating briefly at 80 °C.

Quickly quench hot CHP solution in an ice water bath (~30-90 sec) before using it to bind to unfolded collagen in the tissue.

Apply quenched CHP solution to tissue section. Completely cover tissue section with CHP staining solution and allow overnight binding at 4 °C in a humidity chamber.

Wash sections using 3 x 5-minute washes of 1× PBS or 1× Tween-20.

### Mount coverslips and image.

After washing off excess CHP solution, the tissue can be imaged, and imported into ImageJ/FIJI for fluorescent signal quantification. The signal intensity correlates with the amount of collagen within the sample. As the tissue sections get thicker, they contain more collagen and therefore we expect to see higher signal intensity from CHP binding to denatured collagen strands. As shown in Fig. 4, CHP signal is more intense as the tissue gets thicker, confirming that CHP signal correlates to total collagen content. The intensity was determined by measuring the average pixel intensity of the total area imaged after background subtraction. This method allows researchers to easily visualize and quantify the total collagen content within a tissue section.

### Discussion

Once the appropriate imaging settings are found, CHP staining allows for easy determination of total collagen content in different tissue samples. This is valuable for examining total collagen content in fibrotic tissues, and it may prove beneficial for evaluating wound healing or disease progression as collagen remodeling is associated with numerous pathological disease and healthy organ upkeep. The fluorescent intensity can be quantified using the ImageJ/FIJI platform.

## Claims

1. A method of measuring total collagen content in a sample, comprising
denaturing collagen in the sample by heat to produce a fully denatured sample,
contacting labeled collagen hybridizing peptides (CHPs) to the denatured collagen, and
measuring a signal from the labeled CHPs to determine the total collagen content in the sample.

2. The method according to claim 1, wherein the denaturing comprises heating the sample at a temperature from 60 °C to 160 °C.

3. The method according to claim 1 or 2, further comprising
contacting labeled collagen hybridizing peptides (CHPs) to the sample prior to denaturing collagen, and
measuring a signal from the labeled CHPs to determine the total denatured collagen content in the sample.

4. The method according to any one of the preceding claims, the CHPs are labeled with fluorescent or biotin dyes.

5. The method according to any one of the preceding claims, wherein the sample is a solution (e.g., homogenizing tissue sample, ECM, etc),
and/or wherein the sample is a tissue section (e.g., bone section),
and/or wherein the sample is an artificial tissue section.

6. The method according to claim 5, wherein the tissue section has a thickness of 1-100 µm.

7. The method according to any one of the preceding claims, wherein each of the labeled CHPs comprises a sequence represented by Formula I:
L-Sₘ-(Gly-X-Y)₃₋₂₀ (Formula I)
in which L is one or more detectable moieties; S is a spacer molecule and m is an integer from 0 to 10; Gly is glycine; and at least one of X and Y is proline, modified proline, and/or hydroxyproline.

8. The method according to any one of the preceding claims, wherein each of the CHPs comprises the sequence of any one of SEQ ID NOs: 1-337.

9. The method according to any one of the preceding claims, wherein the sample is from a patient with liver fibrosis.

10. The method according to any one of the preceding claims, wherein the method excludes trypsin-hydroxyproline assay.

11. The method according to any one of the preceding claims, wherein the method excludes picrosirius red with polarized light.

12. The method according to any one of the preceding claims, wherein the method takes less than three days,
or less than two days.

13. A method of determining presence or progression of a condition in a patient, comprising
detecting total collagen content in a sample from the patient by the method according to any one of claims 1-12,
wherein the condition is one or more selected from the group consisting of fibrosis, wound healing, idiopathic pulmonary fibrosis (IPF), aged skin, liver fibrosis, nonalcoholic steatohepatitis (NASH), nonalcoholic fatty liver disease (NAFLD), alcoholic fatty liver disease (AFLD), kidney fibrosis, myocardial infarction (MI), age-related macular degeneration (AMD), osteoarthritis (OA), and keratoconus.

14. The method according to claim 13, wherein the total collagen content and the damaged collagen content are combined as a ratio for an objective measure of damaged collagen that is normalized to the specific sample group,
wherein the ratio is optionally used as predictive biomarker of progression or resolution in a diseased state.

15. The method according to claim 13 or 14, wherein the condition is liver fibrosis, nonalcoholic steatohepatitis (NASH), nonalcoholic fatty liver disease (NAFLD) or alcoholic fatty liver disease (AFLD).

16. The method according to any one of claims 13-15, further comprising detecting total collagen content in another sample from the patient,
and/or further comprising detecting non-triple helical collagen in another sample from the patient by contacting the labeled CHPs to the non-triple helical collagen.

## Patentansprüche

1. Verfahren zur Bestimmung des Gesamtkollagengehalts in einer Probe, umfassend
das Denaturieren des Kollagens in der Probe durch Wärme, um eine vollständig denaturierte Probe zu erhalten,
das In-Kontakt-Bringen von markierten, mit Kollagen hybridisierenden Peptiden (CHPs) mit dem denaturierten Kollagen und
das Messen eines Signals von den markierten CHPs, um den Gesamtkollagengehalt in der Probe zu bestimmen.

2. Verfahren nach Anspruch 1, wobei das Denaturieren das Erwärmen der Probe auf eine Temperatur von 60 °C bis 160 °C umfasst.

3. Verfahren nach Anspruch 1 oder 2, ferner umfassend
das In-Kontakt-Bringen von markierten, mit Kollagen hybridisierenden Peptiden (CHPs) mit der Probe vor der Denaturierung des Kollagens und
das Messen eines Signals von den markierten CHPs, um den Gesamtgehalt an denaturiertem Kollagen in der Probe zu bestimmen.

4. Verfahren nach einem der vorstehenden Ansprüche, wobei die CHPs mit Fluoreszenz- oder Biotin-Farbstoffen markiert sind.

5. Verfahren nach einem der vorstehenden Ansprüche, wobei die Probe eine Lösung (z. B. eine homogenisierte Gewebeprobe, ECM usw.) ist,
und/oder wobei die Probe ein Gewebeschnitt (z. B. ein Knochenschnitt) ist,
und/oder wobei die Probe ein artifizieller Gewebeschnitt ist.

6. Verfahren nach Anspruch 5, wobei der Gewebeschnitt eine Dicke von 1-100 µm aufweist.

7. Verfahren nach einem der vorstehenden Ansprüche, wobei jedes der markierten CHPs eine durch die Formel I dargestellte Sequenz umfasst:
L-Sₘ-(Gly-X-Y)₃₋₂₀ (Formel I)
worin L eine oder mehrere nachweisbare Gruppen ist; S ein Spacermolekül ist und m eine ganze Zahl von 0 bis 10 ist; Gly Glycin ist; und mindestens eines von X und Y Prolin, modifiziertes Prolin und/oder Hydroxyprolin ist.

8. Verfahren nach einem der vorstehenden Ansprüche, wobei jedes der CHPs die Sequenz einer der SEQ ID NOs: 1-337 umfasst.

9. Verfahren nach einem der vorstehenden Ansprüche, wobei die Probe von einem Patienten mit Leberfibrose stammt.

10. Verfahren nach einem der vorstehenden Ansprüche, wobei das Verfahren einen Trypsin-Hydroxyprolin-Assay ausschließt.

11. Verfahren nach einem der vorstehenden Ansprüche, wobei das Verfahren Pikro-Siriusrot-Färbung mit polarisiertem Licht ausschließt.

12. Verfahren nach einem der vorstehenden Ansprüche, wobei das Verfahren weniger als drei Tage oder weniger als zwei Tage in Anspruch nimmt.

13. Verfahren zur Bestimmung des Vorliegens oder des Fortschreitens eines Zustands bei einem Patienten, umfassend
das Bestimmen des Gesamtkollagengehalts in einer Probe des Patienten nach dem Verfahren gemäß einem der Ansprüche 1 bis 12,
wobei es sich bei dem Zustand um eine oder mehrere Zustände handelt, die ausgewählt sind aus der Gruppe bestehend aus Fibrose, Wundheilung, idiopathischer Lungenfibrose (IPF), gealterter Haut, Leberfibrose, nichtalkoholischer Steatohepatitis (NASH), nichtalkoholischer Fettlebererkrankung (NAFLD), alkoholischer Fettlebererkrankung (AFLD), Nierenfibrose, Myokardinfarkt (MI), altersbedingter Makuladegeneration (AMD), Osteoarthritis (OA) und Keratokonus.

14. Verfahren nach Anspruch 13, wobei der Gesamtkollagengehalt und der Gehalt an geschädigtem Kollagen als Verhältnis kombiniert werden, um ein objektives Maß für geschädigtes Kollagen zu erhalten, das auf die jeweilige Probengruppe normalisiert ist, wobei das Verhältnis optional als prädiktiver Biomarker für das Fortschreiten oder die Rückbildung eines Krankheitszustands verwendet wird.

15. Verfahren nach Anspruch 13 oder 14, wobei es sich bei der Erkrankung um Leberfibrose, nichtalkoholische Steatohepatitis (NASH), nichtalkoholische Fettlebererkrankung (NAFLD) oder alkoholische Fettlebererkrankung (AFLD) handelt.

16. Verfahren nach einem der Ansprüche 13 bis 15, das ferner das Bestimmen des Gesamtkollagengehalts in einer weiteren Probe des Patienten umfasst, und/oder ferner das Bestimmen von nicht-dreifach-helikalem Kollagen in einer weiteren Probe des Patienten durch In-Kontakt-Bringen der markierten CHPs mit dem nicht-dreifachhelikalen Kollagen umfasst.

## Revendications

1. Procédé de mesure de la teneur totale en collagène dans un échantillon, comprenant
la dénaturation du collagène dans l'échantillon par la chaleur afin de produire un échantillon totalement dénaturé,
la mise en contact de peptides d'hybridation de collagène (CHPs) marqués et du collagène dénaturé, et
la mesure d'un signal provenant des CHPs marqués afin de déterminer la teneur totale en collagène dans l'échantillon.

2. Procédé selon la revendication 1, dans lequel la dénaturation comprend le chauffage de l'échantillon à une température de 60°C à 160°C.

3. Procédé selon la revendication 1 ou 2, comprenant en outre
la mise en contact de peptides d'hybridation de collagène (CHPs) marqués et de l'échantillon avant la dénaturation du collagène, et
la mesure d'un signal provenant des CHPs marqués afin de déterminer la teneur totale en collagène dénaturé dans l'échantillon.

4. Procédé selon l'une quelconque des revendications précédentes, dans lequel les CHPs sont marqués par des colorants fluorescents ou biotine.

5. Procédé selon l'une quelconque des revendications précédentes, dans lequel l'échantillon est une solution (par exemple, un échantillon de tissu homogénéisé, ECM, etc.),
et/ou dans lequel l'échantillon est une section de tissu (par exemple, une section osseuse),
et/ou dans lequel l'échantillon est une section de tissu artificiel.

6. Procédé selon la revendication 5, dans lequel la section de tissu a une épaisseur de 1 - 100 µm.

7. Procédé selon l'une quelconque des revendications précédentes, dans lequel chacun des CHPs marqué comprend une séquence représentée par la Formule I :
L-Sₘ-(Gly-X-Y)₃₋₂₀ (Formule I)
où L représente un ou plusieurs groupements détectables ; S représente une molécule d'espacement et m représente un nombre entier de 0 à 10 ; Gly représente la glycine ; et au moins un parmi X et Y représente la proline, la proline modifiée et/ou l'hydroxyproline.

8. Procédé selon l'une quelconque des revendications précédentes, dans lequel chacun des CHPs comprend la séquence de l'une quelconque parmi SEQ ID NO : 1 à 337.

9. Procédé selon l'une quelconque des revendications précédentes, dans lequel l'échantillon provient d'un patient atteint de fibrose hépatique.

10. Procédé selon l'une quelconque des revendications précédentes, dans lequel le procédé exclut le dosage de la trypsine-hydroxyproline.

11. Procédé selon l'une quelconque des revendications précédentes, dans lequel le procédé exclut le rouge picrosirius avec lumière polarisée.

12. Procédé selon l'une quelconque des revendications précédentes, dans lequel le procédé prend moins de trois jours,
ou moins de deux jours.

13. Procédé de détermination de la présence ou de la progression d'une affection chez un patient, comprenant
la détection de la teneur totale en collagène dans un échantillon provenant du patient par le procédé selon l'une quelconque des revendications 1 - 12,
dans lequel l'affection est une ou plusieurs affections choisies dans le groupe constitué de la fibrose, de la cicatrisation, de la fibrose pulmonaire idiopathique (FPI), du vieillissement cutané, de la fibrose hépatique, de la stéatohépatite non alcoolique (NASH), de la stéatose hépatique non alcoolique (NAFLD), de la stéatose hépatique alcoolique (AFLD), de la fibrose rénale, de l'infarctus du myocarde (IM), de la dégénérescence maculaire liée à l'âge (DMLA), de l'arthrose (OA) et du kératocône.

14. Procédé selon la revendication 13, dans lequel la teneur totale en collagène et la teneur en collagène endommagé sont combinées sous forme d'un rapport pour une mesure objective du collagène endommagé qui est normalisée par rapport au groupe d'échantillons spécifique,
dans lequel le rapport est éventuellement utilisé comme biomarqueur prédictif de la progression ou de la résolution de l'état pathologique.

15. Procédé selon la revendication 13 ou 14, dans lequel l'affection est la fibrose hépatique, la stéatohépatite non alcoolique (NASH), la stéatose hépatique non alcoolique (NAFLD) ou la stéatose hépatique alcoolique (AFLD).

16. Procédé selon l'une quelconque des revendications 13 - 15, comprenant en outre la détection de la teneur totale en collagène dans un autre échantillon provenant du patient,
et/ou comprenant en outre la détection du collagène non triple hélicoïdal dans un autre échantillon provenant du patient par mise en contact des CHPs marqués et du collagène non triple hélicoïdal.
